# EUROPEAN PATENT APPLICATION

(11) **EP 3 798 219 A1**
(43) Date of publication of application: **31.03.2021**
(21) Application number: 19807918.8
(22) Date of filing: 20.05.2019
(51) Int. Cl.: C07D 471/04, A61P 9/00, A61P 9/10, A61K 31/437

(54) **PHENYL-SUBSTITUTED DIHYDRONAPHTHYRIDINE COMPOUND AND USE THEREOF**

(30) Priority: 22.05.2018 CN 201810497354
(71) Applicant: Sunshine Lake Pharma Co., Ltd., Dongguan, Guangdong 523000 (CN)
(72) Inventor: YANG, Chuanwen, Dongguan, Guangdong 523871 (CN); WANG, Xiaojun, Dongguan, Guangdong 523871 (CN); ZUO, Yinglin, Dongguan, Guangdong 523871 (CN); ZHANG, Yingjun, Dongguan, Guangdong 523871 (CN); WANG, Jiancheng, Dongguan, Guangdong 523871 (CN); WANG, Hui, Dongguan, Guangdong 523871 (CN); CHI, Bo, Dongguan, Guangdong 523871 (CN)
(74) Representative: Hoffmann Eitle
(86) International application number: PCT/CN2019/087516
(87) International publication number: WO 2019/223629

(57) **Abstract**

The present invention relates to a phenyl-substituted dihydronaphthyridine compound and use thereof, and further relates to a pharmaceutical composition comprising the compound. According to the present invention, the compound or the pharmaceutical composition can be used as a mineralocorticoid receptor antagonist.

## Description

### FIELD

The invention belongs to the technical field of medicine, and specifically relates to a phenyl-substituted dihydronaphthyridine compound and use thereof, and further relates to a pharmaceutical composition containing the compound described herein. The compound or the pharmaceutical composition of the present invention can be used as a mineralocorticoid receptor antagonist.

### BACKGROUND

The Mineralocorticoid Receptor (MR) is a nuclear hormone receptor activated by aldosterone, which regulates the expression of many genes involved in electrolyte homeostasis and cardiovascular diseases. The increase in circulating aldosterone increases blood pressure through its effect on urinary sodium excretion, while potentially affecting the brain, heart, and vascular system. In addition, hyperaldosteronism is related to many physiological processes that lead to kidney and cardiovascular diseases. While hyperaldosteronism is commonly caused by aldosterone-producing adenomas, resistant hypertensive patients frequently suffer from increased aldosterone levels, often termed as "Aldosterone Breakthrough", which is due to the increase in serum potassium or residual AT1R activity. Hyperaldosteronism and aldosterone breakthrough typically results in increased MR activity and MR antagonists have been shown to be effective as anti-hypertensive agents and also in the treatment of heart failure and primary hyperaldosteronism. In addition, MR antagonists are also proven effective in preclinical models of kidney disease, and can be combined with standard therapies to reduce proteinuria in patients with kidney disease, such as chronic kidney disease, including diabetic nephropathy.

Aldosterone is a steroid hormone formed in the adrenal cortex. Its production greatly depends on renal blood flow and is indirectly regulated. Any reduction in renal blood flow results in renin in the kidney releasing and entering into the circulating blood. This in turn activates the formation of angiotensin II, which on the one hand has a constrictive effect on arterial blood vessels, but on the other hand also stimulates the formation of aldosterone in the adrenal cortex. Therefore, the kidney is used as a blood pressure sensor in the blood circulation, and indirectly as a volume sensor, and the serious loss of volume is offset by the renin-angiotensin-aldosterone system. This is realized on the one aspect by increasing blood pressure (angiotensin II effect), on the other aspect by increasing the reabsorption of sodium and water in the kidney to rebalance the filling state of the vascular system (aldosterone effect). The control system may be pathologically damaged in various ways. For example, a chronic decrease in renal blood flow (for example, due to heart failure and consequent blood blockage in the venous system) leads to excessive release of aldosterone. This is followed by the expansion of blood volume and increasing the supply of blood volume to the heart, thus leading to the weakness of heart. And obstruction of blood in the lungs, shortness of breath, edema of the extremities, ascites, and pleural effusion may be caused by this; renal blood flow further decreases. In addition, the effect of excessive aldosterone leads to a decrease of the potassium concentration in the blood and extracellular fluid. In myocardium that has been damaged in other ways before, if there is a level below the critical minimum, it may induce arrhythmia with fatal results. This is probably one of the main causes of sudden cardiac death that often occurs in patients with heart failure.

In addition, aldosterone is also believed to be the cause of many of the myocardial remodeling processes commonly observed in heart failure. Therefore, hyperaldosteronism is the key to the pathogenesis and prognosis of heart failure, which is initially induced by various types of injury such as myocardial infarction, myocardial inflammation, or hypertension. This hypothesis has been supported by the following facts: the overall mortality of patients was significantly reduced through the use of aldosterone antagonists in an extensive clinical study of patients with chronic heart failure and acute myocardial infarction (B. Pitt, F. Zannad, W.J. Remme et, al, N. Engl. J. Med. ML 709-717 (1999); B. Pitt, W. Remme, F. Zannad et, al, N. Engl. J. Med 1309-1321 (2003))).

In addition, in visceral tissues, such as the kidney and the gut, MR regulates sodium retention, potassium excretion and water balance in response to aldosterone. MR expression in the brain also appears to play a role in the control of neuronal excitability, in the negative feedback regulation of the hypothalamic-pituitary-adrenal axis, and in the cognitive aspects of behavioral performance (Castren et al, J. of Neuroendocrinology, 3, 461-66 (1993)).

Elevation in aldosterone levels, or excess stimulation of mineralocorticoid receptors, is linked to several physiological disorders or pathologic disease states, including Conn's Syndrome, primary and secondary hyperaldosteronism, increased sodium retention, increased magnesium and potassium excretion (diuresis), increased water retention, hypertension (isolated systolic and combined systolic/diastolic), arrhythmias, myocardial fibrosis, myocardial infarction, Bartter's Syndrome, and disorders associated with excess catecholamine levels. (Hadley, M.E., ENDOCRINOLOGY, 2nd Ed., pp 366-81, (1988); and Brilla et al, Journal of Molecular and Cellular Cardiology, 25 (5), pp 563-75 (1993)). Compounds and/or pharmaceutical compositions which act as MR antagonists may be of value in the treatment of any of the above conditions.

Despite significant therapeutic advances of mineralcorticoid receptor antagonist in the treatment of hypertension and heart failure, the current standard of care is suboptimal and there is a clear unmet medical need for additional therapeutic/pharmacological interventions. This invention addresses those needs by providing compounds and compositions which may be useful for the treatment or prevention of diabetic nephropathy, hypertension, heart failure, other cardiovascular disorders and other aldosterone disorders.

### SUMMARY OF THE INVENTION

The present invention provides a phenyl-substituted dihydronaphthyridine compound having mineralocorticoid receptor (MR) antagonic effect and a pharmaceutical composition thereof, and the use of the compound or the pharmaceutical composition in the manufacture of a medicament for treating, preventing or lessening hyperaldosteronism, diabetic nephropathy, hypertension, heart failure (including chronic heart failure, *etc*.), sequelae of myocardial infarction, liver cirrhosis, renal failure, stroke and other diseases in patients.

In one aspect, provided herein is a compound having Formula (I) or a stereoisomer, a geometric isomer, a tautomer, an *N*-oxide, a hydrate, a solvate, a metabolite, an ester, a pharmaceutically acceptable salt or a prodrug thereof, 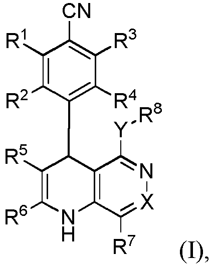
wherein, X is CR^{x} or N;
each of R¹, R², R³ and R⁴ is independently H, D, amino, hydroxy, mercapto, cyano, nitro, C₁₋₆ alkyl, C₁₋₆ alkoxy, C₁₋₆ haloalkyl, C₁₋₆ haloalkoxy, C₁₋₆ alkylamino, carboxy, C₁₋₆ alkanoyl, C₁₋₆ alkylsulfonyl, aminocarbonyl, aminosulfonyl, C₃₋₈ cycloalkyl, C₆₋₁₀ aryl, 3-8 membered heterocyclyl or 5-10 membered heteroaryl;
R⁵ is cyano, -C(=O)R^{c} or -C(=O)NR^{a}R^{b};
R⁶ is H, D, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, 3-8 membered heterocyclyl or 5-10 membered heteroaryl;
each of R⁷ and R^{x} is independently H, D, halogen, cyano, C₁₋₆ alkoxycarbonyl, carboxy, C₁₋₆ alkyl, C₁₋₆ alkoxy, C₁₋₆ haloalkyl, C₁₋₆ haloalkoxy, C₁₋₆ alkylamino, C₁₋₆ alkanoyl, C₁₋₆ alkylsulfonyl, aminocarbonyl or aminosulfonyl;
each of R^{a} and R^{b} is independently H, D, C₁₋₆ alkyl or C₁₋₆ haloalkyl;
R^{c} is H, D, OH, C₁₋₆ alkoxy, C₁₋₆ alkyl, C₁₋₆ haloalkoxy, C₁₋₆ haloalkyl, C₆₋₁₀ aryl or 5-6 membered heteroaryl;
Y is O or S;
R⁸ is C₁₋₆ alkyl, C₃₋₈ cycloalkyl, 3-8 membered heterocyclyl, 5-6 membered heteroaryl, C₃₋₈ cycloalkyl-C₁₋₆-alkyl, (3-8 membered heterocyclyl)-C₁₋₆ alkyl, (5-6 membered heteroaryl)-C₁₋₆ alkyl, phenyl or phenyl C₁₋₆ alkyl; wherein R⁸ is unsubstituted or substituted with 1, 2, 3 or 4 R^{z};
each R^{z} is independently =O, deuterium, fluorine, chlorine, bromine, iodine, hydroxy, cyano, NH₂, C₁₋₆ alkyl, C₁₋₆ alkoxy, C₁₋₆ haloalkoxy, C₁₋₆ haloalkyl, C₁₋₆ alkylamino, C₁₋₆ alkylsulfonyl, C₁₋₆ alkanoyl, C₃₋₈ cycloalkyl, 3-8 membered heterocyclyl, 5-6 membered heteroaryl or C₆₋₁₀ aryl; wherein, each R^{z} is independently unsubstituted or substituted with 1, 2, 3 or 4 R^{w};
each R^{w} is independently =O, deuterium, fluorine, chlorine, bromine, iodine, hydroxy, cyano, NH₂, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkanoyl, C₃₋₈ cycloalkyl, 3-8 membered heterocyclyl, 5-6 membered heteroaryl or C₆₋₁₀ aryl.

In one aspect, the compound having Formula (I) of the present invention is a compound of Formula (Ia) or a compound of Formula (Ib) or a stereoisomer, a geometric isomer, a tautomer, an *N*-oxide, a hydrate, a solvate, a metabolite, an ester, a pharmaceutically acceptable salt or a prodrug thereof, 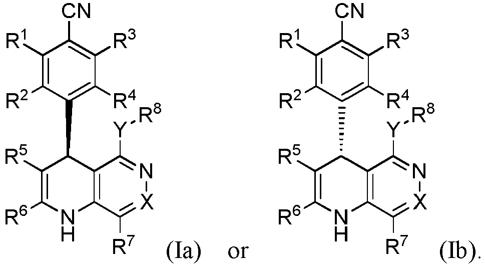 wherein, each of R¹, R², R³, R⁴, R⁵, R⁶, R⁷, R⁸, X and Y is as define herein.

In some embodiments, each of R¹, R², R³ and R⁴ is independently H, deuterium, amino, hydroxy, mercapto, cyano, nitro, C₁₋₄ alkyl, C₁₋₄ alkoxy, C₁₋₄ haloalkyl, C₁₋₄ haloalkoxy, C₁₋₄ alkylamino, carboxy, C₁₋₄ alkanoyl, C₁₋₄ alkylsulfonyl, aminocarbonyl, aminosulfonyl, C₃₋₆ cycloalkyl, C₆₋₁₀ aryl, 3-6 membered heterocyclyl or 5-6 membered heteroaryl;
R⁶ is H, D, C₁₋₄ alkyl, C₁₋₄ alkoxy, C₁₋₄ haloalkyl, C₁₋₄ haloalkoxy, C₁₋₄ alkylamino, C₃₋₆ cycloalkyl, C₆₋₁₀ aryl, 3-6 membered heterocyclyl or 5-6 membered heteroaryl;
each of R⁷ and R^{x} is independently H, D, halogen, cyano, C₁₋₄ alkoxycarbonyl, carboxy, C₁₋₄ alkyl, C₁₋₄ alkoxy, C₁₋₄ haloalkyl, C₁₋₄ haloalkoxy, C₁₋₄ alkylamino, C₁₋₄ alkylcarbonyl, C₁₋₄ alkylsulfonyl, aminocarbonyl or aminosulfonyl;
each of R^{a} and R^{b} is independently H, D, C₁₋₄ alkyl or C₁₋₄ haloalkyl.

In some embodiments, each of R¹, R², R³ and R⁴ is independently H, D, amino, hydroxy, mercapto, cyano, nitro, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino, dimethylamino, carboxy, methylcarbonyl, ethylcarbonyl, methylsulfonyl, aminocarbonyl or aminosulfonyl;
each of R^{a} and R^{b} is independently H, D, methyl, ethyl, propyl, butyl, trifluoromethyl, difluoromethyl, monofluoromethyl, 2,2-difluoroethyl, 1,2-difluoroethyl or trifluoroethyl (including but not limited to 2,2,2-trifluoroethyl);
R⁶ is H, D, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino, dimethylamino, cyclopropyl, cyclobutyl, cyclopentyl, cyclohexyl, phenyl, naphthyl, epoxyethyl, pyrrolidinyl, piperidinyl, piperazinyl, morpholinyl, pyridyl, pyrrolyl, thiazolyl, pyrazolyl or pyrimidinyl;
R⁷ is H, D, cyano, methylcarbonyl, ethylcarbonyl, propylcarbonyl, methoxycarbonyl, ethoxycarbonyl, propoxycarbonyl, carboxy, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino or dimethylamino.

In some embodiments, the compound of the present invention is a compound having Formula (IIIa) or a compound of formula (IIIb), or a stereoisomer, a geometric isomer, a tautomer, an *N*-oxide, a hydrate, a solvate, a metabolite, an ester, a pharmaceutically acceptable salt or a prodrug thereof,

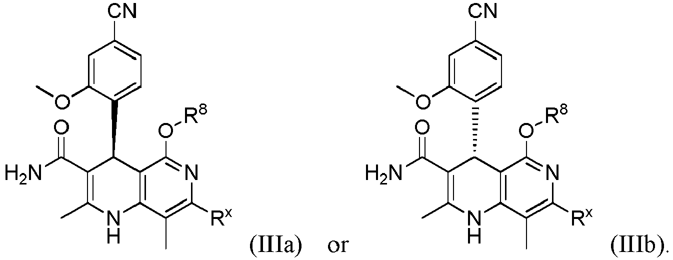

In some embodiments, R^{x} is H, D, cyano, methylcarbonyl, ethylcarbonyl, propylcarbonyl, methoxycarbonyl, ethoxycarbonyl, propoxycarbonyl, carboxy, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino or dimethylamino.

In some embodiments, R⁸ is C₃₋₆ cycloalkyl, 3-6 membered heterocyclyl, 5-6 membered heteroaryl, C₃₋₆ cycloalkyl-C₁₋₄-alkyl, (3-6 membered heterocyclyl)-C₁₋₄ alkyl or (5-6 membered heteroaryl)-C₁₋₄ alkyl; wherein R⁸ is unsubstituted or substituted with 1, 2, 3 or 4 R^{z}; R^{z} is as defined herein.

In other embodiments, R⁸ is cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl, cyclopropylmethyl, cyclopropylethyl, cyclobutylmethyl, cyclobutylethyl, cyclopentylmethyl, cyclopentylethyl, cyclohexylmethyl, cyclohexylethyl, azetidinylmethyl, azetidinylethyl, oxetanylmethyl, oxetanylethyl, pyrrolidylmethyl, pyrrolidylethyl, tetrahydrofurylmethyl, tetrahydrofurylethyl, tetrahydrothienylmethyl, tetrahydrothienylethyl, piperidinylmethyl, piperidinylethyl, piperazinylmethyl, piperazinylethyl, morpholinylmethyl, morpholinylethyl, pyrrolylmethyl, pyrrolylethyl, furylmethyl, furylethyl, thienylmethyl, thienylethyl, thiazolylmethyl, thiazolylethyl, pyrazolylmethyl, pyrazolylethyl, imidazolylmethyl, imidazolylethyl, triazolylmethyl, triazolylethyl, tetrazolylmethyl, tetrazolylethyl, pyridylmethyl, pyridylethyl, pyrimidinylmethyl or pyrimidinylethyl; wherein R⁸ is unsubstituted or substituted with 1, 2, 3 or 4 R^{z}; R^{z} is as defined herein.

In some embodiments, each R^{z} is independently =O, deuterium, fluorine, chlorine, bromine, iodine, hydroxy, cyano, NH₂, C₁₋₄ alkyl, C₁₋₄ alkoxy, C₁₋₄ haloalkoxy, C₁₋₄ haloalkyl, C₁₋₄ alkylamino, C₁₋₄ alkylsulfonyl, C₁₋₄ alkanoyl, C₃₋₆ cycloalkyl, 3-6 membered heterocyclyl, 5-6 membered heteroaryl or C₆₋₁₀ aryl; wherein, each R^{z} is independently unsubstituted or substituted with 1, 2, 3 or 4 R^{w};
each R^{w} is independently =O, deuterium, fluorine, chlorine, bromine, iodine, hydroxy, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkanoyl, C₃₋₆ cycloalkyl, 3-6 membered heterocyclyl, 5-6 membered heteroaryl or C₆₋₁₀ aryl.

In some embodiments, each R^{z} is independently =O, deuterium, fluorine, chlorine, bromine, iodine, hydroxy, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methylethylamino, diethylamino, methylsulfonyl, ethylsulfonyl, methylcarbonyl, ethylcarbonyl, cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl or phenyl; wherein each R^{z} is independently unsubstituted or substituted with 1, 2, 3 or 4 R^{w};

In some embodiments, each R^{w} is independently =O, deuterium, fluorine, chlorine, bromine, iodine, hydroxy, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methylethylamino, diethylamino, methylsulfonyl, ethylsulfonyl, methylcarbonyl, ethylcarbonyl, cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl or phenyl.

In another embodiments, provided herein is a pharmaceutical composition comprising the compound disclosed herein, and optionally, further comprising at least one of pharmaceutically acceptable carrier, excipient, diluent, adjuvant and vehicle.

In some embodiments, the pharmaceutical composition further comprising one or more other active ingredients selected from ACE inhibitors, renin inhibitors, angiotensin II receptor antagonists, β-receptor blockers, acetylsalicylic acid, diuretics, calcium antagonists, statins, digitalis derivatives, calcium sensitizers, nitrates and antithrombotic agents.

In one aspect, provided herein is use of the compound or the composition in the manufacture of a medicament for treating, preventing or lessening the following diseases in a patient: diabetic nephropathy, hyperaldosteronism, hypertension, heart failure, sequelae of myocardial infarction, liver cirrhosis, renal failure or stroke.

In other aspect, provided herein is use of the compound or the pharmaceutical composition disclosed herein in the manufacture a medicament as a mineralocorticoid receptor antagonist.

In one aspect, provided herein is the compound or the composition disclosed herein for use in treating, preventing or lessening the following diseases in a patient: diabetic nephropathy, hyperaldosteronism, hypertension, heart failure, sequelae of myocardial infarction, liver cirrhosis, renal failure or stroke.

In other aspect, provided herein is the compound or the pharmaceutical composition disclosed herein for use in antagonizing mineralocorticoid receptor.

In one aspect, provided herein is a method for treating, preventing or lessening the following diseases in a patient: diabetic nephropathy, hyperaldosteronism, hypertension, heart failure, sequelae of myocardial infarction, liver cirrhosis, renal failure or stroke, comprising administering to the patient the compound or the composition disclosed herein.

In other aspect, provided herein is a method of using the compound or the pharmaceutical composition disclosed herein in antagonizing mineralocorticoid receptor.

### DETAILED DESCRIPTION OF THE INVENTION

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and Smith et al., "March's Advanced Organic Chemistry", John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles used herein refer to one or more than one (*i.e.* at least one) articles of the grammatical objects. By way of example, "a component" means one or more components, and thus, possibly, more than one component is contemplated and may be employed or used in an implementation of the described embodiments.

As used herein, "patient" refers to a human (including adults and children) or other animal.

In some embodiments, "patient" refers to a human.

The term "comprise" is an open expression, it means comprising the contents disclosed herein, but don't exclude other contents.

"Stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, etc.

"Enantiomers" refers to two stereoisomers of a compound which are non-superimposable mirror images of one another.

"Diastereomer" refers to a stereoisomer with two or more centers of chirality and whose molecules are not mirror images of one another. Diastereomers have different physical properties, *e.g*. melting points, boling points, spectral properties or biological activities. Mixture of diastereomers may separate under high resolution analytical procedures such as electrophoresis and chromatography such as HPLC.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Any asymmetric atom (*e.g*., carbon or the like) of the compound(s) disclosed herein can be present in racemic or enantiomerically enriched, for example the (*R*)-, (*S*)- or (*R,S*)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration.

Any resulting mixtures of stereoisomers can be separated on the basis of the physicochemical differences of the constituents, into the pure or substantially pure geometric isomers, enantiomers, diastereomers, for example, by chromatography and/or fractional crystallization.

The term "tautomer" or "tautomeric form" refers to structural isomers of different energies which are interconvertible via a low energy barrier. Where tautomerization is possible (*e.g*. in solution), a chemical equilibrium of tautomers can be reached. For example, protontautomers (also known as prototropic tautomers) include interconversions via migration of a proton, such as keto-enol and imine-enamine isomerizations. Valence tautomers include interconversions by reorganization of some of the bonding electrons. A specific example of keto-enol tautomerization is the interconversion of pentane-2,4-dione and 4-hydroxypent-3-en-2-one tautomers. Another example of tautomerization is phenol-keto tautomerization. The specific example of phenol-keto tautomerisms is pyridin-4-ol and pyridin-4(1*H*)-one tautomerism. Unless otherwise stated, all tautomeric forms of the compounds disclosed herein are within the scope of the invention.

As described herein, compounds disclosed herein may optionally be substituted with one or more substituents, such as are illustrated generally below, or as exemplified by particular classes, subclasses, and species of the invention.

Furthermore, what need to be explained is that the phrase "each... is independently" and "each of... and... is independently", unless otherwise stated, should be broadly understood. The specific options expressed by the same symbol are independent of each other in different groups; or the specific options expressed by the same symbol are independent of each other in same groups. Similarly, the "independently" in the description "...independently and optionally" should also be interpreted in the above-mentioned broad sense.

The term "optional" or "optionally" refers to that a subsequently described event or circumstance may but need not occur, and that the description includes instances where the event or circumstance occurs and instances in which it does not.

At each part of the present specification, substitutes of compounds disclosed herein are disclosed in groups or in ranges. It is specifically intended that the invention includes each and every individual subcombination of the members of such groups and ranges. "C₁-C₆ alkyl" or "C₁₋₆ alkyl" refers to independently disclosed methyl, ethyl, C₃ alkyl, C₄ alkyl, C₅ alkyl and C₆ alkyl; "C₁₋₄ alkyl " specifically refers to independently disclosed methyl, ethyl, C₃ alkyl (*i.e.,* propyl, including *n*-propyl and *i*-propyl) and C₄ alkyl (*i.e.,* including *n*-butyl, *i*-butyl, s-butyl and *t*-butyl).

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "alkyl" or "alkyl group" refers to a saturated linear or branched-chain monovalent hydrocarbon group of 1 to 20 carbon atoms, wherein the alkyl group is optionally substituted with one or more substituents described herein. In some embodiments, the alkyl group contains 1-12 carbon atoms. In other embodiments, the alkyl group contains 1-6 carbon atoms, *i.e.,* C₁₋₆ alkyl. In still other embodiments, the alkyl group contains 1-4 carbon atoms, *i.e.,* C₁₋₄ alkyl. In yet other embodiments, the alkyl group contains 1-3 carbon atoms, *i.e.,* C₁₋₃ alkyl. In some embodiments, the C₁₋₆ alkyl described in the invention contains C₁₋₄ alkyl; In other embodiments, the C₁₋₆ alkyl described in the invention contains C₁₋₃ alkyl.

Examples of the alkyl group include, but are not limited to, methyl, ethyl, propyl (including *n*-propyl and isopropyl), butyl (including *n*-butyl, isobutyl, *sec*-butyl, *tert*-butyl), *n*-pentyl, 2-pentyl, 3-pentyl, 2-methyl-2-butyl, 3-methyl-2-butyl, 3-methyl-1-butyl, 2-methyl-1-butyl, *n*-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, *n*-heptyl, *n*-octyl, and the like.

The term "alkoxy" refers to an alkyl group, as previously defined, attached to parent molecular moiety via an oxygen atom. Some non-limiting examples of the alkoxy group include methoxy, ethoxy, propoxy (including 1-propoxy or 2-propoxy), butoxy (including *n*-butyl, *i*-butyl, s-butyoxy, *t*-butoxy), *etc.*

The terms "haloalkyl" or "haloalkoxy" refer to alkyl or alkoxy substituted with one or more halogen atoms. Some non-limiting examples of "haloalkyl" or "haloalkoxy" groups include trifluoromethyl, trifluoromethoxy, chloroethyl (*e.g*., 2-chloroethyl), trifluoroethyl (including, but not limited to 2,2,2-trifluoroethyl), 2,2-difluoroethyl, 2-chloro-1-methylethyl, and the like.

The term "amino" refers to group -NH₂. The term "carboxy" refers to group -COOH. The term "hydroxy", "cyano", "nitro", "mercapto" respectively refers to group -OH, -CN, -NO₂, -SH. The term "oxo" refers to group =O.

The term "alkylamino" refers to -NH₂ group substituted with one or two alkyl, *i.e.,* the alkylamino described herein contains monoalkylamino and dialkylamino; wherein the alkyl is as defined herein. Some non-limiting examples of alkylamino group include, but are not limited to, methylamino, ethylamino, methylethylamino, dimethylamino, and the like.

The term "cycloalkyl" refers to a saturated ring having 3 to 12 ring carbon atoms as a monocyclic, bicyclic, or tricyclic ring system. In some embodiments, the cycloalkyl contains 3 to 10 ring carbon atoms, for example, C₃₋₁₀ cycloalkyl. In still other embodiments, the cycloalkyl contains 3 to 8 ring carbon atoms, for example, C₃₋₈ cycloalkyl. In yet other embodiments, the cycloalkyl contains 3 to 6 ring carbon atoms, for example, C₃₋₆ cycloalkyl. Examples of cycloalkyl groups include, but are not limited to, cyclopropyl, cyclobutyl, cyclopentyl, cyclohexyl, cycloheptyl, cyclooctyl, and the like. Wherein, as described herein, C₃₋₈ cycloalkyl includes C₃₋₆ cycloalkyl; the C₃₋₆ cycloalkyl includes cyclopropyl, cyclobutyl, cyclopentyl and cyclohexyl. The cycloalkyl group may be optionally substituted with one or more substituents disclosed herein.

The term "heterocyclyl" refers to a saturated or partial unsaturated monocyclic, bicyclic or tricyclic ring system having 3-12 ring atoms in which at least one ring atom is selected from nitrogen, sulfur and oxygen; wherein the heterocyclyl is nonaromatic, and doesn't contain any aromatic ring. Unless otherwise specified, the heterocyclyl group may be carbon or nitrogen linked, and a -CH₂- group can be optionally replaced by a -C(=O)- group. In which, the sulfur can be optionally oxygenized to *S*-oxide and the nitrogen can be optionally oxygenized to *N*-oxide. The term "heterocyclyl" can be used interchangeably with the term "heterocyclic" or "heterocyclic ring". Examples of heterocyclyl groups include, but are not limited to, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl or morpholinyl, *etc.* As described in the present invention, the heterocyclyl group may consists of 3-8 atoms or 3-6 atoms, wherein the atom is C, N, O or S and at least one atom is N, O or S; wherein, the heterocyclyl group consisting of 3-8 atoms includes a heterocyclyl group consisting of 3-6 atoms; the heterocyclyl group consisting of 3-6 atoms includes a heterocyclyl group consisting of 3-5 atoms; Specifically, the heterocyclyl group consisting of 3-6 atoms includes, but is not limited to, oxiranyl 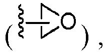 aziridinyl 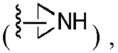 azetidinyl 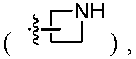 oxetanyl 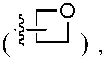 pyrrolidinyl 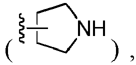 tetrahydrofuranyl 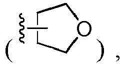 tetrahydrothienyl 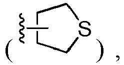 thiazolidyl 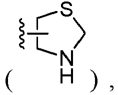 pyrazolidyl 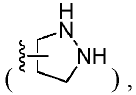 pyrazolinyl 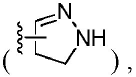 oxazolidinyl 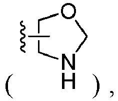 imidazolidyl 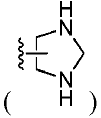 , piperidyl 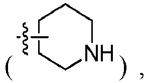 piperazinyl 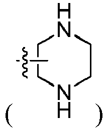 or morpholinyl 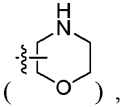 and the like. The heterocyclyl group may be optionally substituted with one or more substituents disclosed herein.

The term "halogen" refers to fluorine (F), chlorine (Cl), bromine (Br) or iodine (I).

The term "aryl" refers to monocyclic, bicyclic and tricyclic carbocyclic ring systems having a total of six to fourteen ring members, or six to twelve ring members, or six to ten ring members, wherein at least one ring in the system is aromatic, and the aryl group has a single point or multipoint of attachment to the rest of the molecule. The term "aryl" and "aromatic ring" can be used interchangeably herein. Some non-limiting examples of the aryl group include phenyl, 2,3-dihydro-1*H*-indenyl, naphthalenyl and anthracenyl, *etc.* The aryl group may be optionally substituted with one or more substituents disclosed herein. Unless otherwise specified, the group "C₆₋₁₀ aryl" means an aryl group containing 6-10 ring carbon atoms.

The term "heteroaryl" refers to monocyclic, bicyclic and tricyclic ring systems having a total of 5 to 12 ring members, or 5 to 10 ring members, or 5 to 6 ring atoms, wherein at least one ring is aromatic, and in which at least one ring contains 1, 2, 3 or 4 ring heteroatoms, and the heteroaryl group has a single point or multipoint of attachment to the rest of the molecule. When a -CH₂- group is present in the heteroaryl group, the -CH₂- group can be optionally replaced by -C(=O)-. Unless otherwise specified, the heteroaryl group can be connected to the rest of the molecule (such as the main structure in the general formula) through any reasonable position (which may be C in CH or N in NH). The term "hetreroaryl" and "heteroaromatic ring" or "heteroaromatic compound" can be used interchangeably herein. Examples of heteroaryl groups include, but are not limited to, furyl, imidazolyl, isoxazolyl, oxazolyl, pyrrolyl, pyrazolyl, pyridyl, pyrimidinyl, pyridazinyl, pyrazinyl, thienyl, thiazolyl, triazolyl, tetrazolyl, *etc.* The heteroaryl group may be optionally substituted with one or more substituents disclosed herein. In some embodiments, the heteroaryl group is a 5-10 membered heteroaryl group, refers that the heteroaryl group contains 1-9 ring carbon atoms and 1, 2, 3, or 4 heteroatoms selected from O, S and N; in other embodiments, the heteroaryl group is a 5-6 membered heteroaryl group, refers that the heteroaryl group contains 1-5 ring carbon atoms and 1, 2, 3, or 4 ring heteroatoms selected from O, S and N. Examples of 5-6 membered heteroaryl group include, but are not limited to, furyl, imidazolyl, isoxazolyl, oxazolyl, pyrrolyl, pyrazolyl, pyridyl, pyrimidinyl, pyridazinyl, pyrazinyl, thienyl, thiazolyl and the like.

The term "j-k membered (each of j and k is independently any non-zero natural number, and k>j)" means that the cyclic group consists of j-k ring atoms, and the ring atoms include carbon atoms and/or heteroatoms such as O, N, S, P, *etc*; the "j-k" includes j, k and any natural number between them. For example, "3-8 membered ", "5-10 membered" or "5-6 membered" refers that the cyclic group consists of 3-8, 5-10 or 5-6 ring atoms, the ring atoms include carbon atoms and/or O, N, S, P and other heteroatoms.

The "3-8 membered heterocyclyl" or "3-6 membered heterocyclyl" in the present invention refers to a heterocyclyl group consisting of 3-8 ring atoms or 3-6 ring atoms. Wherein the heterocyclic group contains 1, 2, 3 or 4 heteroatoms selected from N, O, S, and the CH₂ in the heterocyclyl group can be further oxidized to form C(=O). Similarly, S or N in the heterocyclyl group can also be further oxidized to form S(=O), S(=O)₂ or N(=O).

The "5-10 membered heteroaryl" or "5-6 membered heteroaryl" in the present invention refers to a heteroaryl group consisting of 5-10 ring atoms or 5-6 ring atoms, wherein the heteroaryl group contains 1, 2, 3 or 4 heteroatoms selected from N, O and S. In some embodiments, specific examples of "5-10 membered heteroaryl" or "5-6 membered heteroaryl" in the present invention include, but are not limited to, pyridyl, pyrimidinyl, pyrazinyl, thienyl, thiazolyl, furyl, pyrrolyl, pyrazolyl, imidazolyl, triazolyl, *etc.*

The term "alkanoyl" or "alkylcarbonyl" refers to the group -C(=O)-alkyl, wherein the alkyl group is as described in the present invention. Examples of the alkylcarbonyl include, but are not limited to, methylcarbonyl (-C(=O)CH₃), ethylcarbonyl (-C(=O)CH₂CH₃), and so on.

The term "alkoxycarbonyl" refers to the group -C(=O)-R, wherein R is a alkoxy group, the alkoxy group is as described in the present invention. Examples of such group include, but are not limited to, methoxycarbonyl (-C(=O)OCH₃), ethoxycarbonyl (-C(=O)OCH₂CH₃), and so on.

The term " alkyl sulfonyl" refers to the group -S(=O)₂-alkyl, wherein the alkyl group is as described in the present invention. Examples of the alkylsulfonyl include, but are not limited to, methylsulfonyl (-S(=O)₂CH₃), ethylsulfonyl (-S(=O)₂CH₂CH₃), and so on.

The term "aminosulfonyl" refers to group -S(=O)₂NH₂; the term "aminocarbonyl" refers to group -C(=O)NH₂.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solutions (such as saline solutions, aqueous dextrose and glycerol solutions) are preferably employed as carriers, particularly for injectable solutions. Suitable pharmaceutical carriers are described in "Remington's Pharmaceutical Sciences" by E. W. Martin.

The term "prodrug" refers to a compound that is transformed in vivo into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic (C₁₋₂₄) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345, all of which are incorporated herein by reference in their entireties.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salts" refers to organic or inorganic salts of a compound disclosed herein. Pharmaceutically acceptable salts are well known in the art. For example, S. M. Berge et al., describe pharmaceutically acceptable salts in detail in J. Pharmaceutical Sciences, 1977, 66: 1-19, which is incorporated herein by reference. Some non-limiting examples of pharmaceutically acceptable and nontoxic salt formed by acid includes, but is not limited to, inorganic acid salts, such as hydrochloric acid, hydrobromic acid, phosphoric acid, sulfuric acid and perchloric acid; organic acid salts, such as acetic acid, oxalic acid, maleic acid, tartaric acid, citric acid, succinic acid and malonic acid, or by using other methods used in the art such as ion exchange. Other pharmaceutically acceptable salts include adipate, alginate, ascorbate, aspartate, benzenesulfonate, benzoate, bisulfate, borate, butyrate, camphanic acid salt, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, *p*-toluenesulfonate, undecanoate, valerate salts, and the like. Salts derived from appropriate bases include alkali metal, alkaline earth metal, ammonium and N⁺(C₁₋₄ alkyl)₄ salts. This invention also envisions the quaternization of any basic nitrogen-containing groups of the compounds disclosed herein. Water or oil soluble or dispersable products may be obtained by such quaternization. Alkali metals or alkaline earth metals that can form salts include sodium, lithium, potassium, calcium, magnesium, and the like. Further pharmaceutically acceptable salts include appropriate and nontoxic ammonium, quaternary ammonium, and amine cations formed using counterions, such as halide, hydroxide, carboxylate, sulfate, phosphate, nitrate, C₁₋₈ sulfonate or aryl sulfonate.

The term "solvate" refers to an association or complex of one or more solvent molecules and a compound disclosed herein. Examples of solvents that form solvates include, but are not limited to, water, isopropanol, ethanol, methanol, DMSO, ethyl acetate, acetic acid and ethanolamine. The term "hydrate" refers to the complex where the solvent molecule is water.

An "ester" refers to an in vivo hydrolysable ester of a compound containing hydroxy group or carboxy group. for example, a pharmaceutically acceptable ester which is hydrolysed in the human or animal body to produce the parent alcohol or acid. The compound of formula (I) of the present invention contains a carboxy group, which can form a hydrolyzable ester in vivo with an appropriate group. Such groups include, but are not limited to, alkyl, arylalkyl and the like.

An "*N*-oxide" refers to one or more than one nitrogen atoms oxidised to form an *N*-oxide, where a compound contains several amine functions. Particular examples of *N*-oxides are the *N*-oxides of a tertiary amine or a nitrogen atom of a nitrogen-containing heterocycle. *N*-oxides can be formed by treatment of the corresponding amine with an oxidizing agent such as hydrogen peroxide or a per-acid (*e.g.* a peroxycarboxylic acid) (See, Advanced Organic Chemistiy, by Jerry March, 4th Edition, Wiley Interscience, pages). More particularly, *N*-oxides can be made by the procedure of L. W. Deady (Syn. Comm. 1977, 7, 509-514) in which the amine compound is reacted with *m*-chloroperoxybenzoic acid (MCPBA), for example, in an inert solvent such as dichloromethane.

The "compounds of the present invention", "compounds described in the present invention", "compounds described herein" or similar expressions used in the present invention refer to compounds represented by any general structure of the present invention. For example, the compound of the present invention may refer to the compound represented by Formula (I) or Formula (Ia) or Formula (Ib) or Formula (II) or Formula (IIa) or Formula (IIb) or Formula (III) or Formula (IIIa) or Formula (IIIb) in the present invention. The compound of the present invention also includes the specific compound in any one of the examples.

As used herein, the term "treat", "treating" or "treatment" of any disease or disorder refers in one embodiment, to ameliorating the disease or disorder (*i.e.,* slowing or arresting or reducing the development of the disease or at least one of the clinical symptoms thereof). In another embodiment, "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g.,* stabilization of a discernible symptom), physiologically, (*e.g.,* stabilization of a physical parameter), or both. In yet another embodiment, "treat", "treating" or "treatment" refers to preventing or delaying the onset or development or progression of the disease or disorder.

Any formula given herein is also intended to represent isotopically unenriched forms as well as isotopically enriched forms of the compounds. Isotopically enriched compounds have the structure depicted by the general formula given herein, except that one or more atoms are replaced by the atom having a selected atomic mass or mass number. Examples of isotopes that can be incorporated into compounds of the invention include isotopes of hydrogen, carbon, nitrogen, oxygen, phosphorous, sulfur, fluorine, and chlorine, such as ²H (deuterium, D), ³H, ¹¹C, ¹³C, ¹⁴C, ¹⁵N, ¹⁷O, ¹⁸O, ¹⁸F, ³¹P, ³²P, ³⁵S, ³⁶Cl and ¹²⁵I, respectively.

Further, substitution with heavier isotopes, particularly deuterium (*i.e.,* ²H or D) may afford certain therapeutic advantages resulting from greater metabolic stability. For example increased in vivo half-life or reduced dosage requirements or an improvement in therapeutic index. It is understood that deuterium in this context is regarded as a substituent of a compound of Formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). Pharmaceutically acceptable solvates in accordance with the invention include those wherein the solvent of crystallization may be isotopically substituted, e.g. D₂O, acetone-*d₆*, DMSO-*d₆*.

Unless otherwise stated, all tautomeric forms of the compounds disclosed herein are within the scope of the invention. Additionally, unless otherwise stated, structures depicted herein are also meant to include compounds that differ only in the presence of one or more isotopically enriched atoms.

As used herein, the abbreviations for any protective groups, amino acids and other compounds are, unless otherwise indicated, in accord with their common usage, recognized abbreviations, or the IUPAC-IUB Commission on Biochemical Nomenclature (See, Biochem. 1972, 11: 942-944).

### DESCRIPTION OF COMPOUNDS OF THE INVENTION

The present invention provides a phenyl-substituted dihydronaphthyridine compound that can competitively antagonize mineralocorticoid receptor (MR) and a pharmaceutical composition thereof, and the use of the compound or the pharmaceutical composition in the manufacture of a medicament for treating, preventing or lessening diabetic nephropathy, hyperaldosteronism, hypertension, heart failure (including chronic heart failure), sequelae of myocardial infarction, liver cirrhosis, renal failure, stroke and other diseases in patients.

In one aspect, provided herein is a compound having Formula (I) or a stereoisomer, a geometric isomer, a tautomer, an *N*-oxide, a hydrate, a solvate, a metabolite, an ester, a pharmaceutically acceptable salt or a prodrug thereof, 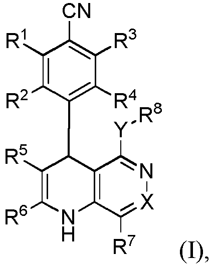 wherein, each of R¹, R², R³, R⁴, R⁵, R⁶, R⁷, R⁸, X and Y is as define herein.

In one aspect, the compound described in the invention is a compound of Formula (Ia) or a compound of Formula (Ib) or a stereoisomer, a geometric isomer, a tautomer, an *N*-oxide, a hydrate, a solvate, a metabolite, an ester, a pharmaceutically acceptable salt or a prodrug thereof, 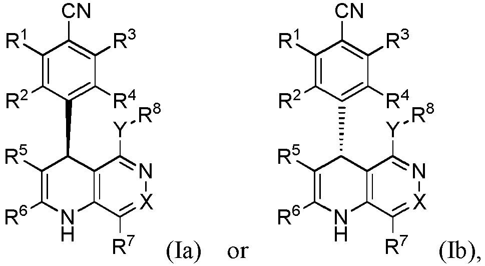 wherein, each of R¹, R², R³, R⁴, R⁵, R⁶, R⁷, R⁸, X and Y is as define herein.

In some embodiments, R⁵ is cyano (-CN), -C(=O)R^{c} or -C(=O)NR^{a}R^{b}, wherein R^{a}, R^{b} and R^{c} are as described in the present invention.

In some embodiments, each of R^{a} and R^{b} is independently H, D, C₁₋₆ alkyl or C₁₋₆ haloalkyl.

In some embodiments, each of R^{a} and R^{b} is independently H, D, C₁₋₄ alkyl or C₁₋₄ haloalkyl.

In some embodiments, each of R^{a} and R^{b} is independently H, D, methyl, ethyl, propyl, butyl, trifluoromethyl, difluoromethyl, monofluoromethyl, 2,2-difluoroethyl, 1,2-difluoroethyl or trifluoroethyl (including but not limited to 2,2,2-trifluoroethyl).

In some embodiments, R^{c} is H, D, OH, C₁₋₆ alkoxy, C₁₋₆ alkyl, C₁₋₆ haloalkoxy, C₁₋₆ haloalkyl, C₆₋₁₀ aryl or 5-6 membered heteroaryl. In some embodiments, R^{c} is H, D, OH, methoxy, ethoxy, propoxy, methyl, ethyl, propyl, butyl, trifluoromethoxy, difluoromethoxy, trifluoromethyl, difluoromethyl, monofluoromethyl, phenyl, pyrrolyl, pyrazolyl, imidazolyl, thienyl, furyl, pyridyl, pyrimidinyl or oxazolyl.

In some embodiments, each of R¹, R², R³ and R⁴ described in the invention is independently H, D, amino, hydroxy, mercapto, cyano, nitro, C₁₋₆ alkyl, C₁₋₆ alkoxy, C₁₋₆ haloalkyl, C₁₋₆ haloalkoxy, C₁₋₆ alkylamino, carboxy, C₁₋₆ alkanoyl, C₁₋₆ alkylsulfonyl, aminocarbonyl, aminosulfonyl, C₃₋₈ cycloalkyl, C₆₋₁₀ aryl, 3-8 membered heterocyclyl or 5-10 membered heteroaryl.

In some embodiments, each of R¹, R², R³ and R⁴ is independently H, D, amino, hydroxy, mercapto, cyano, nitro, C₁₋₄ alkyl, C₁₋₄ alkoxy, C₁₋₄ haloalkyl, C₁₋₄ haloalkoxy, C₁₋₄ alkylamino, carboxy, C₁₋₄ alkanoyl, C₁₋₄ alkylsulfonyl, aminocarbonyl, aminosulfonyl, C₃₋₆ cycloalkyl, C₆₋₁₀ aryl, 3-6 membered heterocyclyl or 5-6 membered heteroaryl.

In some embodiments, each of R¹, R², R³ and R⁴ is independently H, D, amino, hydroxy, mercapto, cyano, nitro, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino, dimethylamino, carboxy, methylcarbonyl, ethylcarbonyl, methylsulfonyl, aminocarbonyl or aminosulfonyl.

In some embodiment, R⁶ is H, D, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, 3-8 membered heterocyclyl or 5-10 membered heteroaryl.

In some embodiment, R⁶ is H, D, C₁₋₄ alkyl, C₁₋₄ alkoxy, C₁₋₄ haloalkyl, C₁₋₄ haloalkoxy, C₁₋₄ alkylamino, C₃₋₆ cycloalkyl, C₆₋₁₀ aryl, 3-6 membered heterocyclyl or 5-6 membered heteroaryl.

In some embodiments, R⁶ is H, D, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino, dimethylamino, cyclopropyl, cyclobutyl , cyclopentyl, cyclohexyl, phenyl, naphthyl, epoxyethyl, pyrrolidyl, piperidyl, piperazinyl, morpholinyl, pyridyl, pyrrolyl, thiazolyl, pyrazolyl or pyrimidinyl.

In one aspect, the compound of Formula (I) described in the invention can be a compound of Formula (II) or a compound of Formula (IIa) or a compound of Formula (IIb) or a stereoisomer, a geometric isomer, a tautomer, an *N*-oxide, a hydrate, a solvate, a metabolite, an ester, a pharmaceutically acceptable salt or a prodrug thereof, 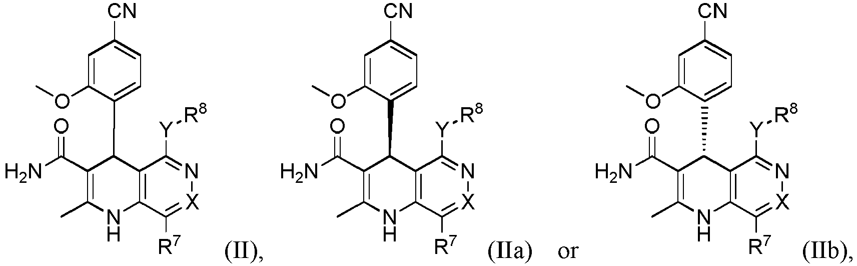 wherein, R⁷, R⁸, X and Y are as described in this invention.

In some embodiments, X in the present invention is CR^{x} or N, wherein, R^{x} has the meaning as described in the present invention.

In some embodiments, R⁷ described in the invention is H, D, halogen, cyano, C₁₋₆ alkoxycarbonyl, carboxy, C₁₋₆ alkyl, C₁₋₆ alkoxy, C₁₋₆ haloalkyl, C₁₋₆ haloalkoxy, C₁₋₆ alkylamino, C₁₋₆ alkanoyl, C₁₋₆ alkylsulfonyl, aminocarbonyl or aminosulfonyl.

In some embodiments, R⁷ described in the invention is H, D, halogen, cyano, C₁₋₄ alkoxycarbonyl, carboxy, C₁₋₄ alkyl, C₁₋₄ alkoxy, C₁₋₄ haloalkyl, C₁₋₄ haloalkoxy, C₁₋₄ alkylamino, C₁₋₄ alkanoyl, C₁₋₄ alkylsulfonyl, aminocarbonyl or aminosulfonyl.

In some embodiments, R⁷ described in the invention is H, D, cyano, methylcarbonyl, ethylcarbonyl, propylcarbonyl, methoxycarbonyl, ethoxycarbonyl, propoxycarbonyl, carboxy, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino or dimethylamino.

In some embodiments, Y is O or S.

In some embodiments, the compound of the invention is a compound of Formula (III) or a stereoisomer, a geometric isomer, a tautomer, an *N*-oxide, a hydrate, a solvate, a metabolite, an ester, a pharmaceutically acceptable salt or a prodrug thereof, 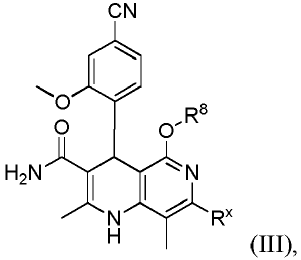 wherein, R⁸ and R^{x} are as described in this invention.

In some embodiments, the compound of the invention is a compound of Formula (IIIa) or a stereoisomer, a geometric isomer, a tautomer, an *N*-oxide, a hydrate, a solvate, a metabolite, an ester, a pharmaceutically acceptable salt or a prodrug thereof, 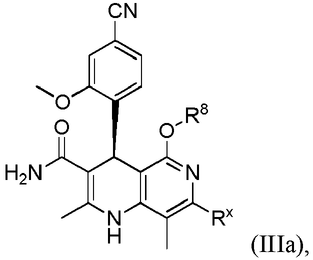 wherein, R⁸ and R^{x} are as described in this invention.

In some embodiments, the compound of the invention is a compound of Formula (IIIb) or a stereoisomer, a geometric isomer, a tautomer, an *N*-oxide, a hydrate, a solvate, a metabolite, an ester, a pharmaceutically acceptable salt or a prodrug thereof, 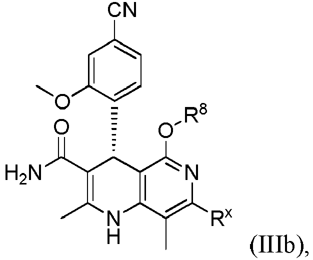 wherein, R⁸ and R^{x} are as described in this invention.

In some embodiments, R^{x} is H, D, halogen, cyano, C₁₋₆ alkoxycarbonyl, carboxy, C₁₋₆ alkyl, C₁₋₆ alkoxy, C₁₋₆ haloalkyl, C₁₋₆ haloalkoxy, C₁₋₆ alkylamino, C₁₋₆ alkanoyl, C₁₋₆ alkylsulfonyl, aminocarbonyl or aminosulfonyl.

In some embodiments, R^{x} described in the invention is H, D, halogen, cyano, C₁₋₄ alkoxycarbonyl, carboxy, C₁₋₄ alkyl, C₁₋₄ alkoxy, C₁₋₄ haloalkyl, C₁₋₄ haloalkoxy, C₁₋₄ alkylamino, C₁₋₄ alkanoyl, C₁₋₄ alkylsulfonyl, aminocarbonyl or aminosulfonyl.

In some embodiments, R^{x} is H, D, cyano, methylcarbonyl, ethylcarbonyl, propylcarbonyl, methoxycarbonyl, ethoxycarbonyl, propoxycarbonyl, carboxy, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl (including but not limited to 2,2,2-trifluoroethyl), trifluoromethoxy, difluoromethoxy, monofluoromethoxy, methylamino or dimethylamino.

In some embodiments, R⁸ described in the invention is C₃₋₈ cycloalkyl, 3-8 membered heterocyclyl, 5-6 membered heteroaryl, C₃₋₈ cycloalkyl-C₁₋₆-alkyl, (3-8 membered heterocyclyl)-C₁₋₆ alkyl, (5-6 membered heteroaryl)-C₁₋₆ alkyl, phenyl or phenyl C₁₋₆ alkyl; wherein R⁸ is unsubstituted or substituted with 1, 2, 3 or 4 R^{z}; R^{z} is as defined herein.

In some embodiments, R⁸ is C₃₋₆ cycloalkyl, 3-6 membered heterocyclyl, 5-6 membered heteroaryl, C₃₋₆ cycloalkyl-C₁₋₄-alkyl, (3-6 membered heterocyclyl)-C₁₋₄ alkyl or (5-6 membered heteroaryl)-C₁₋₄ alkyl; wherein R⁸ is unsubstituted or substituted with 1, 2, 3 or 4 R^{z}; R^{z} is as defined herein.

In some embodiments, R⁸ is cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl, cyclopropylmethyl, cyclopropylethyl, cyclobutylmethyl, cyclobutylethyl, cyclopentylmethyl, cyclopentylethyl, cyclohexylmethyl, cyclohexylethyl, azetidinylmethyl, azetidinylethyl, oxetanylmethyl, oxetanylethyl, pyrrolidylmethyl, pyrrolidylethyl, tetrahydrofurylmethyl, tetrahydrofurylethyl, tetrahydrothienylmethyl, tetrahydrothienylethyl, piperidinylmethyl, piperidinylethyl, piperazinylmethyl, piperazinylethyl, morpholinylmethyl, morpholinylethyl, pyrrolylmethyl, pyrrolylethyl, furylmethyl, furylethyl, thienylmethyl, thienylethyl, thiazolylmethyl, thiazolylethyl, pyrazolylmethyl, pyrazolylethyl, imidazolylmethyl, imidazolylethyl, triazolylmethyl, triazolylethyl, tetrazolylmethyl, tetrazolylethyl, pyridylmethyl, pyridylethyl, pyrimidinylmethyl or pyrimidinylethyl; wherein R⁸ is unsubstituted or substituted with 1, 2, 3 or 4 R^{z}; wherein R^{z} is as defined herein.

In some embodiments, each R^{z} described in the invention is independently =O, deuterium, fluorine, chlorine, bromine, iodine, hydroxy, cyano, NH₂, C₁₋₆ alkyl, C₁₋₆ alkoxy, C₁₋₆ haloalkoxy, C₁₋₆ haloalkyl, C₁₋₆ alkylamino, C₁₋₆ alkylsulfonyl, C₁₋₆ alkanoyl, C₃₋₈ cycloalkyl, 3-8 membered heterocyclyl, 5-6 membered heteroaryl or C₆₋₁₀ aryl; wherein, each R^{z} is independently unsubstituted or substituted with 1, 2, 3 or 4 R^{w}; wherein R^{w} is as defined herein.

In some embodiments, each R^{z} is independently =O, deuterium, fluorine, chlorine, bromine, iodine, hydroxy, cyano, NH₂, C₁₋₄ alkyl, C₁₋₄ alkoxy, C₁₋₄ haloalkoxy, C₁₋₄ haloalkyl, C₁₋₄ alkylamino, C₁₋₄-alkylsulfonyl, C₁₋₄ alkanoyl, C₃₋₆ cycloalkyl, 3-6 membered heterocyclyl, 5-6 membered heteroaryl or C₆₋₁₀ aryl; wherein, each R^{z} is independently unsubstituted or substituted with 1, 2, 3 or 4 R^{w}; wherein R^{w} is as defined herein.

In some embodiments, each R^{z} is independently =O, deuterium, fluorine, chlorine, bromine, iodine, hydroxy, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino , ethylamino, dimethylamino, methylethylamino, diethylamino, methylsulfonyl, ethylsulfonyl, methylcarbonyl, ethylcarbonyl, cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl or phenyl; wherein each R^{z} is independently unsubstituted or substituted with 1, 2, 3 or 4 R^{w}; wherein R^{w} is as defined herein.

In some embodiments, each R^{w} described in the invention is independently =O, deuterium, fluorine, chlorine, bromine, iodine, hydroxy, cyano, NH₂, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkanoyl, C₃₋₈ cycloalkyl, 3-8 membered heterocyclyl, 5-6 membered heteroaryl or C₆₋₁₀ aryl.

In some embodiments, each R^{w} described in the invention is independently =O, deuterium, fluorine, chlorine, bromine, iodine, hydroxy, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkanoyl, C₃₋₆ cycloalkyl, 3-6 membered heterocyclyl, 5-6 membered heteroaryl or C₆₋₁₀ aryl.

In some embodiments, each R^{w} is independently =O, deuterium, fluorine, chlorine, bromine, iodine, hydroxy, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methylethylamino, diethylamino, methylsulfonyl, ethylsulfonyl, methylcarbonyl, ethylcarbonyl, cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl or phenyl.

In some embodiments, R⁸ described in the invention can be but not limited to one of the following groups:

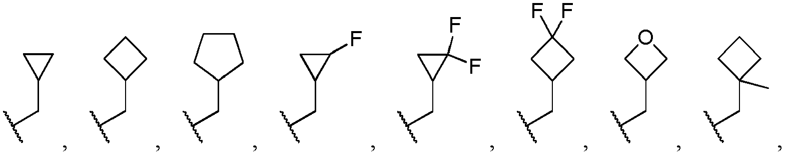

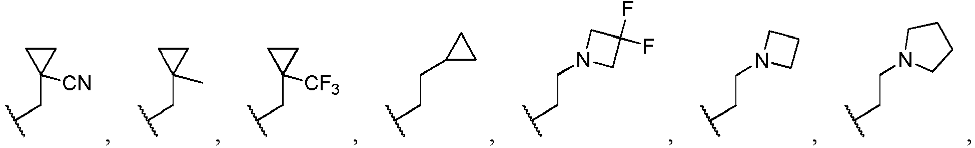

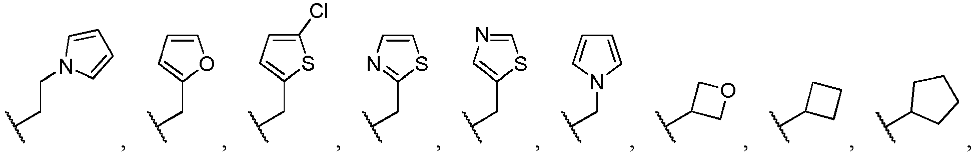

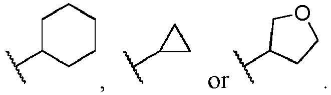

In some embodiments, the compound described in the invention has one of the following structures, or a stereoisomer, a geometric isomer, a tautomer, an *N*-oxide, a hydrate, a solvate, a metabolite, an ester, a pharmaceutically acceptable salt or a prodrug thereof,

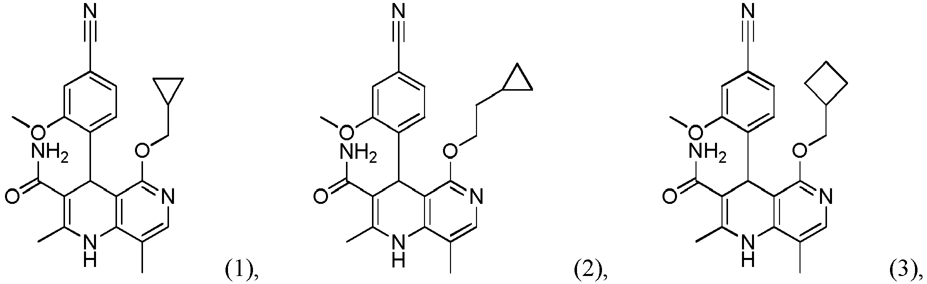

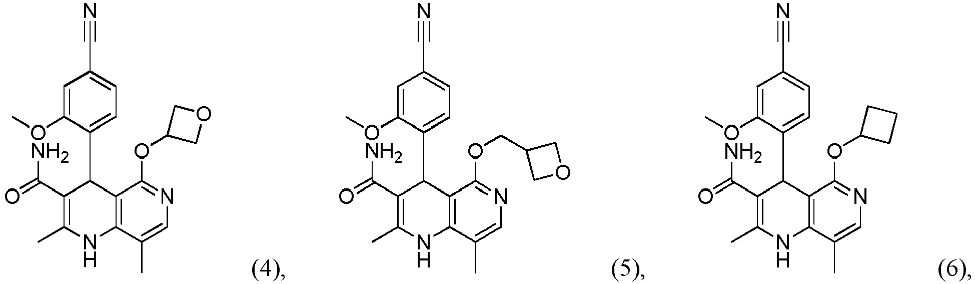

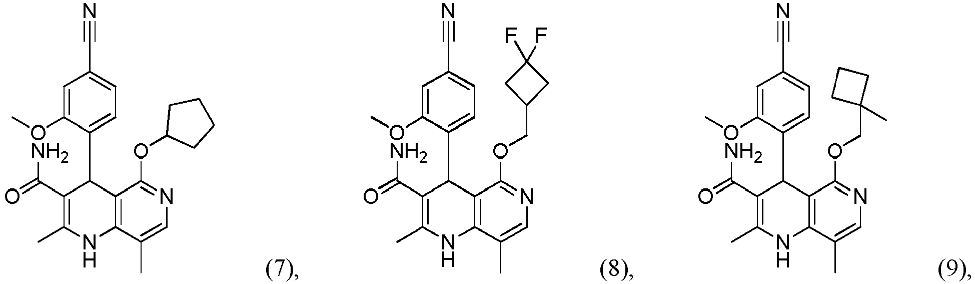

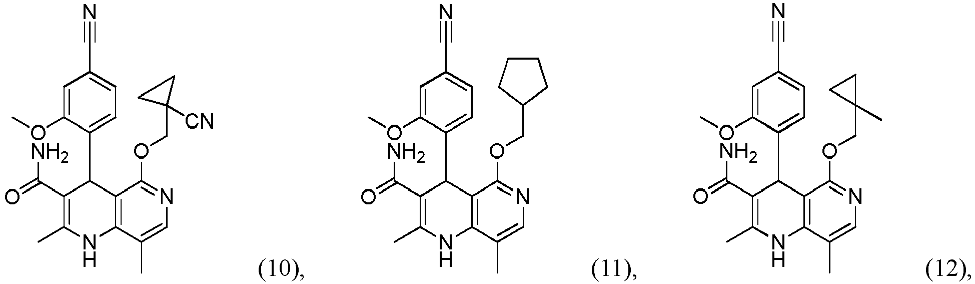

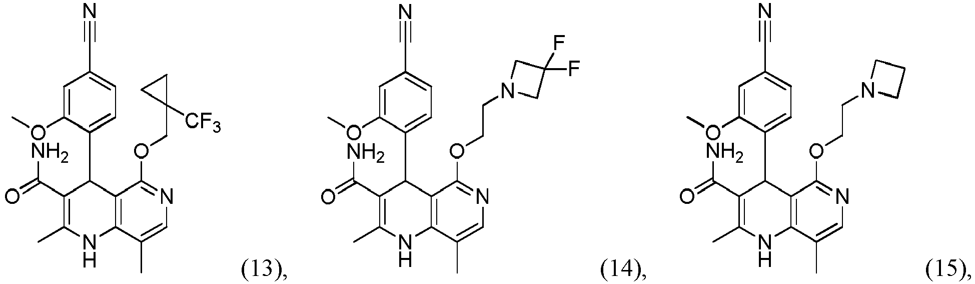

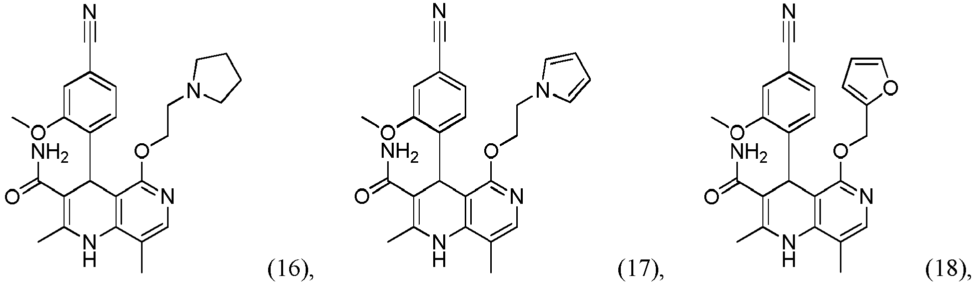

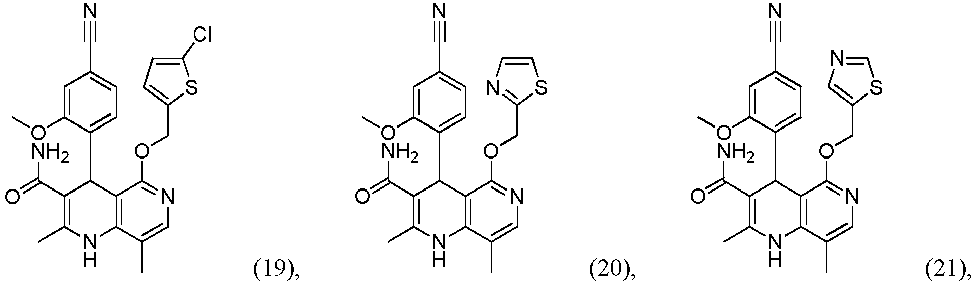

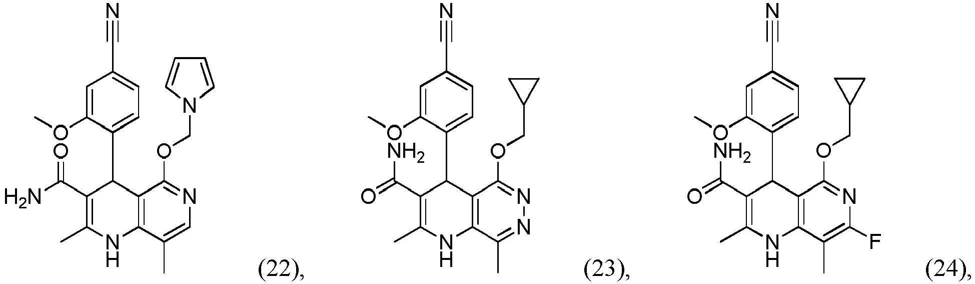

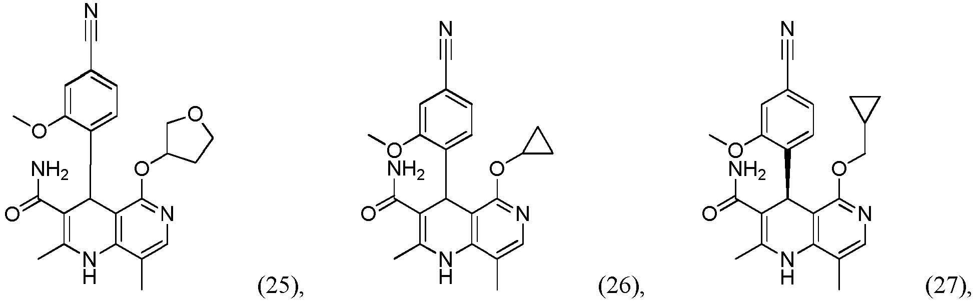

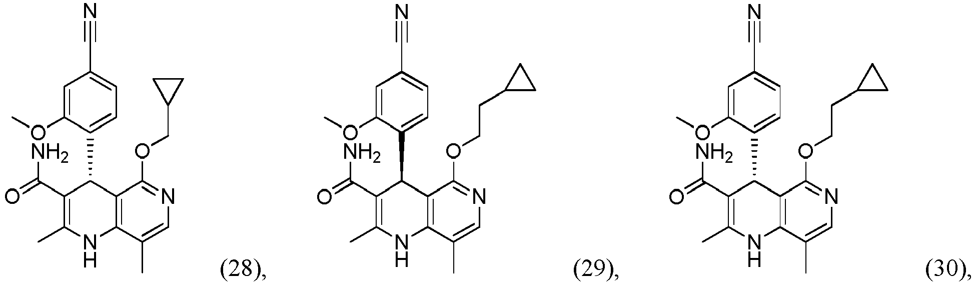

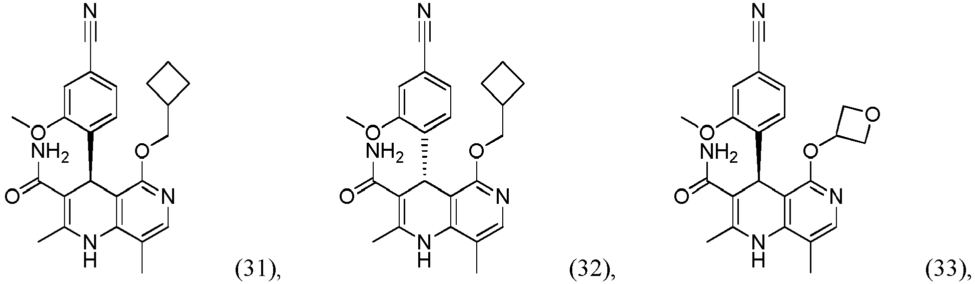

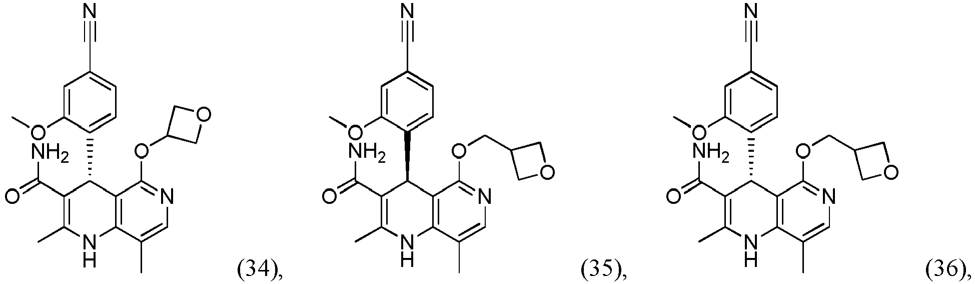

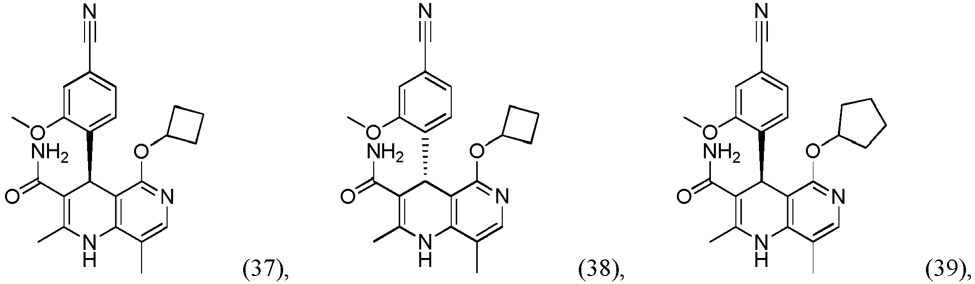

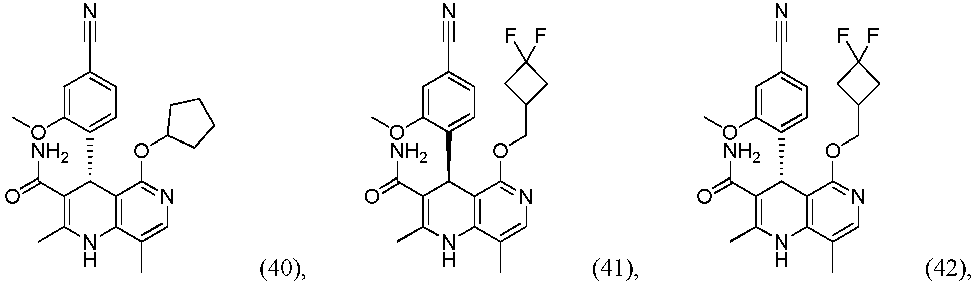

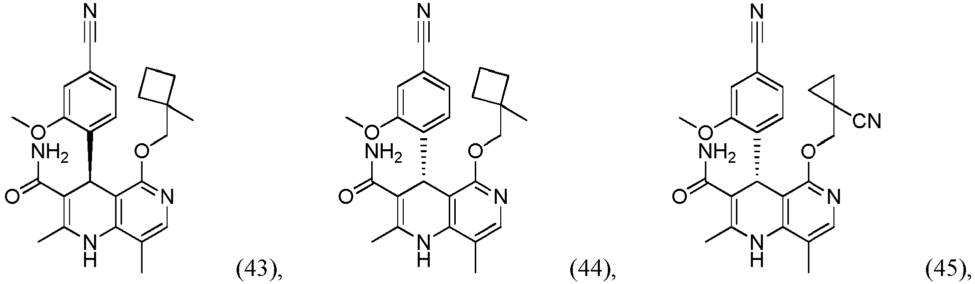

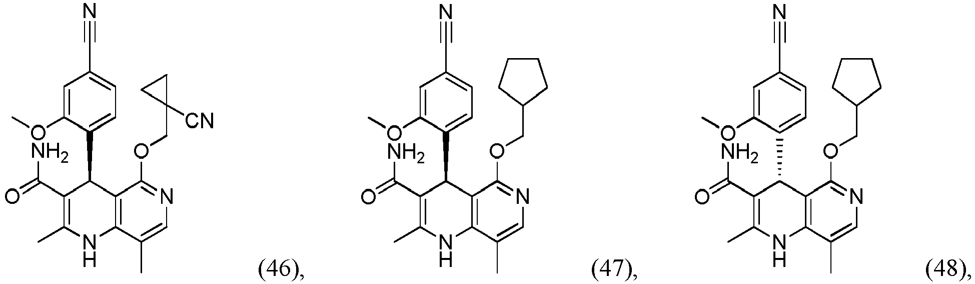

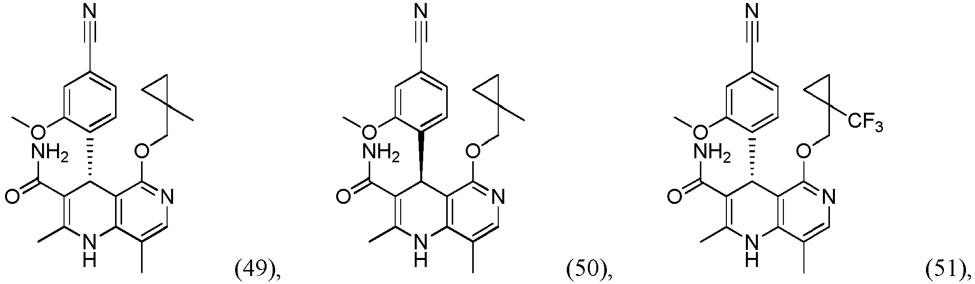

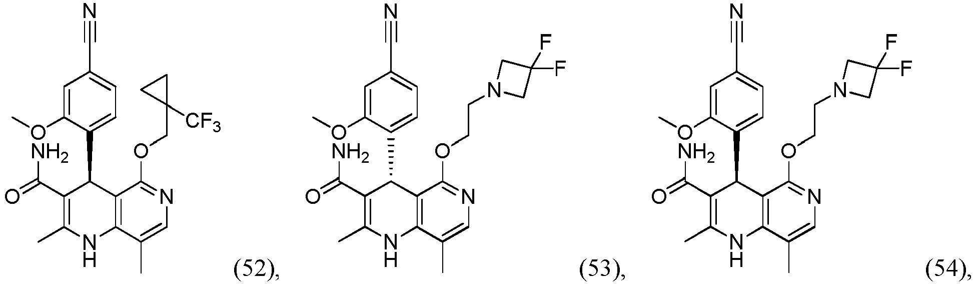

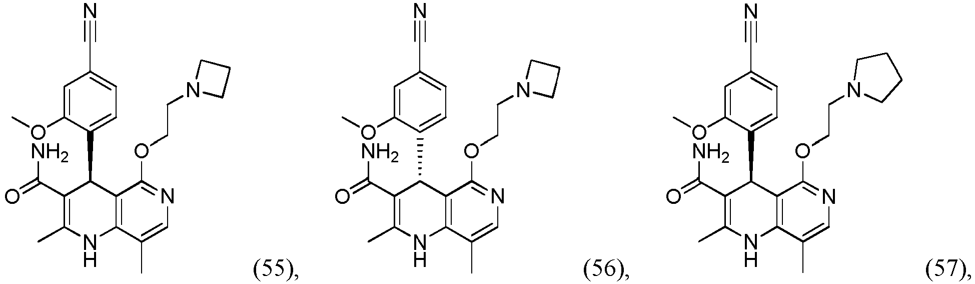

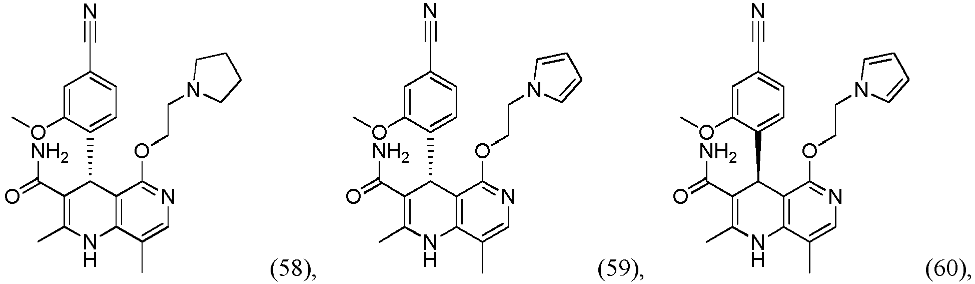

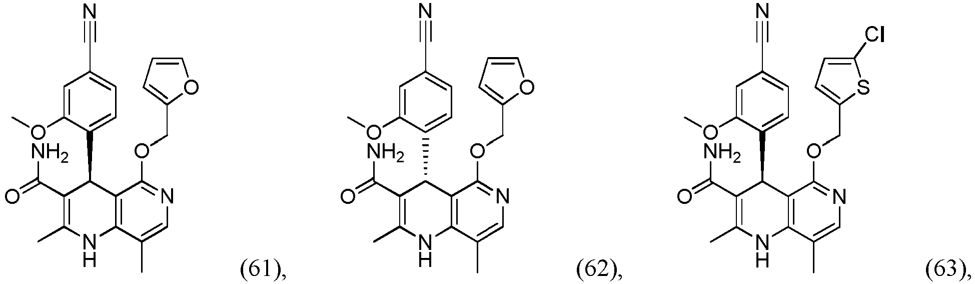

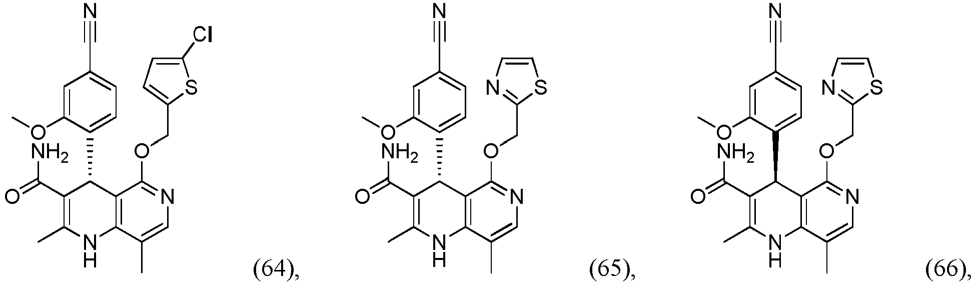

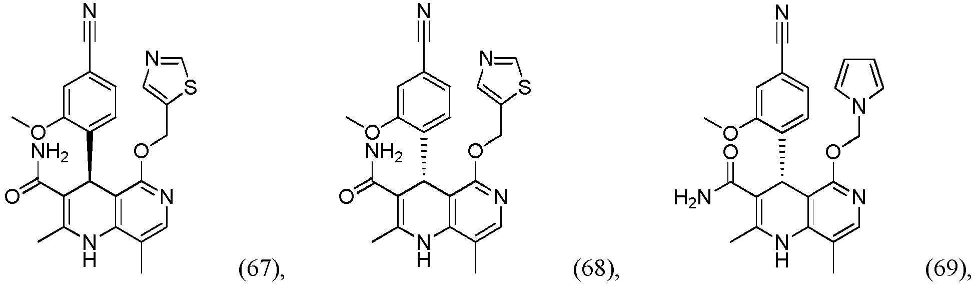

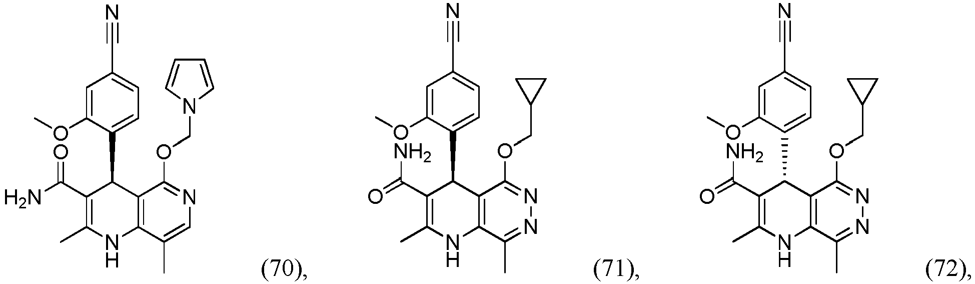

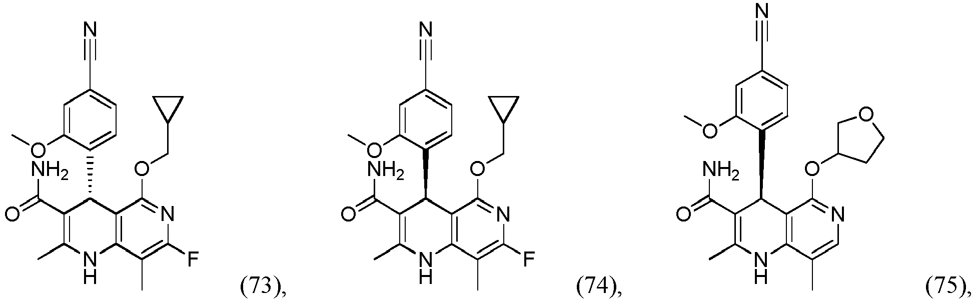

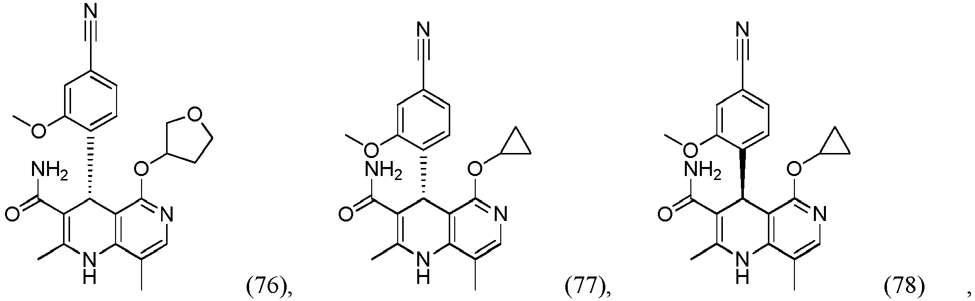

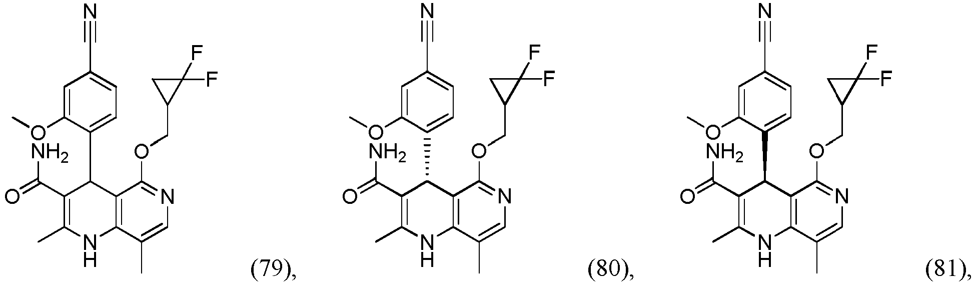

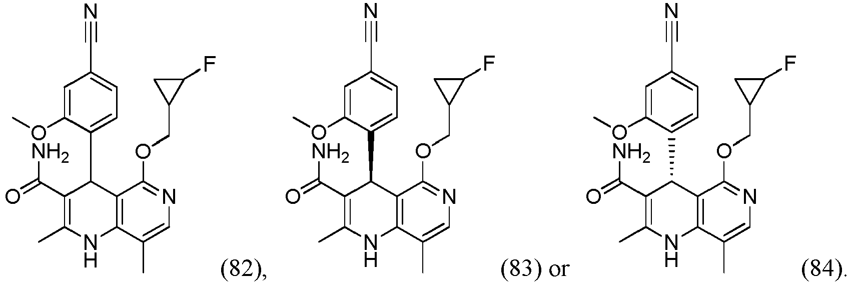

In other aspect, provided herein is a pharmaceutical composition comprising the compound disclosed herein.

In some embodiments, the pharmaceutical composition disclosed herein optionally further comprises pharmaceutically acceptable carriers, excipients, diluents, adjuvants, or combination thereof.

In some embodiments, the pharmaceutical composition further comprising one or more other active ingredients selected from ACE inhibitors, renin inhibitors, angiotensin II receptor antagonists, β-receptor blockers, acetylsalicylic acid, diuretics, calcium antagonists, statins, digitalis derivatives, calcium sensitizers, nitrates and antithrombotic agents.

In one aspect, provided herein is use of the compound or the composition in the manufacture of a medicament for treating, preventing or lessening the following diseases in a patient: diabetic nephropathy, hyperaldosteronism, high blood pressure, heart failure (including chronic cardiac failure, and the like), sequelae of myocardial infarction, liver cirrhosis, renal failure or stroke.

In other aspect, also provided herein is use of the compound or the pharmaceutical composition disclosed herein in the manufacture a medicament as a mineralocorticoid receptor antagonist.

In one aspect, provided herein is the compound or the composition for use in treating, preventing or lessening the following diseases in a patient: diabetic nephropathy, hyperaldosteronism, high blood pressure, heart failure (including chronic cardiac failure, and the like), sequelae of myocardial infarction, liver cirrhosis, renal failure or stroke.

In other aspect, provided herein is the compound or the pharmaceutical composition disclosed herein for use in antagonizing the halocorticoid receptor.

In one aspect, provided herein is a method of treating, preventing or lessening the following diseases in a patient: diabetic nephropathy, hyperaldosteronism, high blood pressure, heart failure (including chronic cardiac failure, and the like), sequelae of myocardial infarction, liver cirrhosis, renal failure or stroke, comprising administering to the patient a therapeutically effective amount of the compound or composition.

In other aspect, provided herein is a method of using the compound or the pharmaceutical composition disclosed herein in antagonizing mineralocorticoid receptor, comprising contacting the compound or pharmaceutical composition of the present invention in an effective dose with biological body (including in vivo or in vitro).

The compound or pharmaceutical composition of the present invention competitively antagonize the aldosterone receptor (MR), and therefore they may be useful agents for the treatment and prevention of conditions associated with increased aldosterone levels.

The compound or pharmaceutical composition of the present invention can be used to treat or prevent aldosterone receptor-mediated diseases. The present invention also includes a method of treating or lessening aldosterone receptor-mediated diseases or susceptibility to these conditions in a patient, comprising administering to the patient a therapeutically effective amount of the compound or pharmaceutical composition of the present invention.

The present invention also comprises uses of the compound and pharmaceutically acceptable salts thereof in the manufacture of a medicament for treating mineralocorticoid receptor or aldosterone related diseases in a patient, including those diseases described in the invention. The present invention includes a pharmaceutical composition that includes an effective therapeutic amount of the compound of Formula (I) required for the combination of the compound of Formula (I) and at least one pharmaceutically acceptable carrier, excipient, diluent, adjuvant, and vehicle.

Unless otherwise stated, all hydrates, solvates and pharmaceutically acceptable salts of the compounds disclosed herein are within the scope of the invention.

In certain embodiments, the salt is a pharmaceutically acceptable salt. The phrase "pharmaceutically acceptable" refers to that the substance or composition must be compatible chemically and/or toxicologically, with the other ingredients comprising a formulation, and/or the mammal being treated therewith.

The salts of the compound of the present invention also include salts of the compounds which are not necessarily pharmaceutically acceptable salts, and which may be useful as intermediates for preparing and/or purifying compounds of Formula (I), Formula (Ia) or Formula (Ib) or Formula (II) or Formula (IIa) or Formula (IIb) or Formula (III) or Formula (IIIa) or Formula (IIIb), and/or for separating enantiomers of compounds of Formula (I) or Formula (Ia) or Formula (Ib) or Formula (II) or Formula (IIa) or Formula (IIb) or Formula (III) or Formula ( IIIa) or Formula (IIIb).

The salt of the compound may be prepared by any suitable method available in the art, for example, treatment of the free base with an inorganic acid, such as hydrochloric acid, hydrobromic acid, sulfuric acid, nitric acid, phosphoric acid, and the like. Or with an organic acid, such as acetic acid, maleic acid, succinic acid, mandelic acid, fumaric acid, malonic acid, pyruvic acid, oxalic acid, glycolic acid and salicylic acid; a pyranosidyl acid, such as glucuronic acid and galacturonic acid; an alpha-hydroxy acid, such as citric acid and tartaric acid; an amino acid, such as aspartic acid and glutamic acid; an aromatic acid, such as benzoic acid and cinnamic acid; a sulfonic acid, such as *p*-toluenesulfonic acid, ethanesulfonic acid and the like.

The biological activity of the compounds of the present invention can be assessed by using any conventionally known methods. The detection method is well known in the art. For example, the MR antagonistic activity, pharmacokinetic activity and/or liver microsomal stability of the compound of the present invention can be tested by appropriate conventional methods. The detection method provided by the present invention is presented only as an example and does not limit the present invention. The compound of the present invention is active in at least one of the detection methods provided by the present invention. For example, the compound of the present invention has good antagonistic activity against aldosterone receptors, and has good in vivo pharmacokinetic properties, such as better absorption and exposure, and higher bioavailability; another example, the compound of the present invention has low side effects.

### PHARMACEUTICAL COMPOSITIONS, FORMULATIONS, ADMINISTRATION AND USES OF THE COMPOUNDS OF THE PRESENT INVENTION

According to a further aspect, the pharmaceutical composition of the invention comprises the phenyl-substituted dihydronaphthyridine compound of Formula (I) or Formula (Ia) or Formula (Ib) or Formula (II) or Formula (IIa) or Formula (IIb) or Formula (III) or Formula (IIIa) or Formula (IIIb), the compounds listed herein, or the compounds of Examples 1-10, and a pharmaceutically acceptable carrier, adjuvant, or excipient. The amount of the compound in the composition of the present invention can effectively treat or alleviate the mineralocorticoid receptor or aldosterone-related diseases in the patient.

As described above, the pharmaceutical acceptable compositions disclosed herein further comprise a pharmaceutically acceptable carrier, an adjuvant, or a vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. As described in the following: In Remington: Troy et al., Remington: The Science and Practice of Pharmacy, 21st ed., 2005, Lippincott Williams & Wilkins, Philadelphia, and Swarbrick et al., Encyclopedia of Pharmaceutical Technology, eds. 19881999, Marcel Dekker, New York, both of which are herein incorporated by reference in their entireties, discloses various carriers used in formulating pharmaceutically acceptable compositions and known techniques for the preparation thereof. Except insofar as any conventional carrier medium incompatible with the compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants.

The pharmaceutical composition of the present invention can be administered directly or in the form of a pharmaceutical composition or drug with a suitable carrier or excipient, which is well known in the art. The treatment method of the present invention may comprise administering an effective compound of the present invention to an individual in need. In some embodiments, the individual is a mammalian individual, and in some preferred embodiments, the individual is a human individual.

The effective amount of the compound, pharmaceutical composition or drug of the present invention can be easily determined by routine experiments, and the most effective and convenient route of administration and the most appropriate formulation can also be determined by routine experiments.

The pharmaceutical dosage form of the compound of the present invention can be provided in the form of an immediate release, controlled release, sustained release or target drug release system. For example, commonly used dosage forms include solutions and suspensions, (micro) emulsions, ointments, gels and patches, liposomes, tablets, dragees, soft or hard shell capsules, suppositories, ovules, implants, amorphous or crystalline powder, aerosol and freeze-dried formulations. Depending on the route of administration, special devices may be required to administer or give the drug, such as syringes and needles, inhalers, pumps, injection pens, applicators, or special flasks. Pharmaceutical dosage forms often consist of drugs, excipients, and container/sealing systems. One or more excipients (also called inactive ingredients) can be added into the compound of the present invention to improve or promote the manufacture, stability, administration and safety of the drug, and can provide the desired drug release curve method. Therefore, the types of excipients added to the drug may depend on various factors, such as the physical and chemical properties of the drug, the route of administration, and the preparation steps. Pharmaceutical excipients exist in this field and include those listed in various pharmacopoeias. (See US Pharmacopeia (USP), Japanese Pharmacopoeia (JP), European Pharmacopoeia (EP) and British pharmacopoeia (BP); the U.S. Food and Drug Administration (www.fda.gov), Publications of Center for Drug Evaluation and Research (CEDR), such as "Inactive Ingredient Guide" (1996); "Handbook of Pharmaceutical Additives"(2002, Synapse Information Resources, Inc., Endicott NY; etc.).

The pharmaceutical dosage form of the compound of the present invention can be manufactured by any method well known in the art, for example, by conventional mixing, sieving, dissolving, melting, granulating, making sugar-coated pills, pressing, suspending, squeezing, spray drying, grinding, emulsification, (nano/micron) encapsulation, encapsulation or freeze-drying process. As mentioned above, the composition of the present invention may include one or more physiologically acceptable inactive ingredients, which can facilitate the processing of active molecules into preparations for medical use.

The appropriate formulation depends on the desired route of administration. For example, for intravenous injection, the composition can be formulated in an aqueous solution, if necessary, using physiologically compatible buffers, including, for example, phosphate, histidine or citrate used to adjust the pH of the formulation, and tonicity agent, such as sodium chloride or dextrose. For transmucosal or nasal administration, semi-solid, liquid formulations or patches may be preferred, and may contain penetration enhancers; such penetration agents are generally known in the art. For oral administration, the compounds can be formulated into liquid or solid dosage forms and used as immediate release or controlled/sustained release formulations. Suitable dosage forms for oral ingestion by individuals include tablets, pills, dragees, hard and soft shell capsules, liquids, gels, syrups, ointments, suspensions, and emulsions. The compounds may also be formulated in rectal compositions such as suppositories or retention enemas, for example containing conventional suppository bases such as cocoa butter or other glycerides.

The compounds of the invention can act systemically and/or locally. They can be administered in a suitable manner, for example, by oral administration, gastrointestinal administration, pulmonary administration, nasal administration, sublingual administration, translingual administration, buccal administration, rectal administration, dermal administration. transdermal administration, conjunctival administration, ear canal administration or as a graft or stent. The compounds of the invention are preferably administered orally or parenterally.

Suitable modes of oral administration are as follows: according to the working methods of the prior art, rapid release and/or improved methods of release of the compounds of the present invention, including crystalline and/or amorphous and/or dissolved form of the compound of the present invention, for example, a tablet (uncoated tablet or, for example, a tablet coated with a gastric juice resistant or delayed dissolving coating or insoluble coating that controls the release of the compound of the present invention), a tablet or film/flake that rapidly disintegrating in the oral cavity, a film/lyophilized body, a capsule (such as hard or soft capsules), sugar-coated tablet, granule, pill, powder, emulsion, suspension, aerosol or solution.

For other routes of administration, suitable examples are inhaled drug forms (including powder inhalers, sprays), nasal drops, solutions or sprays, tablets for tongue, sublingual or buccal administration, films/ flakes or capsules, suppositories, ear or eye preparations, vaginal capsules, aqueous suspensions (lotions, shock mixtures), lipophilic suspensions, ointments, creams, transdermal therapeutic systems (*e.g*. patches), emulsions (Milch), paste, foam, spray powder, implant or stent.

The therapeutically effective amount of the compound of the present invention should be present in the above-mentioned pharmaceutical preparation at a concentration of about 0.1 to 99.5%, preferably about 0.5 to 95% by weight of the entire mixture. The therapeutically effective dose can first be estimated using various methods well known in the art. The initial dose for animal studies can be based on the effective concentration established in the cell culture assay. The dosage range suitable for a human individual can be determined, for example, by data obtained from animal studies and cell culture assays. In certain embodiments, the compound of the present invention can be prepared as a medicament for oral administration. An exemplary dose of the compound of the present invention in a medicament for oral administration is from about 0.01 to about 100 mg/kg (where kg represents the weight of the subject). In some embodiments, the medicament comprises from about 0.01 to about 20 mg/kg (where kg represents the weight of the subject), or optionally from about 0.01 to about 10 mg/kg (where kg represents the weight of the subject), or optionally from about 0.01 to about 5.0 mg/kg (where kg represents the weight of the subject). In certain embodiments, the compound of the present invention is administered enterally, and its effective dosage is about 0.001-1 mg/kg, preferably about 0.01-0.5 mg/kg body weight.

The dosage regimen of agents commonly used for oral administration is three times a week, twice a week, once a week, three times a day, twice a day, or once a day. In some embodiments, the compound of the present invention is administered as an active ingredient in a total amount of about 0.001 to about 50, preferably 0.001 to 10 mg/kg body weight per 24 hours. In order to obtain the desired result, optionally, multiple single doses can be used for administration. A single dose may preferably contain the compound of the invention in an amount of about 0.001 to about 30, especially 0.001 to 3 mg/kg body weight.

The effective amount or therapeutically effective amount or dose of an agent (such as the compound of the present) is the amount that bring about improvement in symptoms or prolonged survival of an individual. The toxicity and therapeutic efficacy of the molecule can be determined by standard medical procedures in cell cultures or laboratory animals, for example by measuring LD₅₀ (lethal dose to 50% of the population) and ED₅₀ (the dose that is therapeutically effective for 50% of the population). The dose ratio of toxic effect to therapeutic effect is the therapeutic index, which can be expressed as LD₅₀/ED₅₀. A drug showing a high therapeutic index is preferred.

The effective amount or therapeutically effective amount is the amount of a compound or pharmaceutical composition that will trigger a biological or medical response of a tissue, system, animal or human being explored by researchers, veterinarians, doctors, or other clinicians. The dosage is preferably within a range of circulating concentrations that include the ED₅₀ with little or no toxicity. The dosage can vary within this range, depending on the dosage form used and/or the route of administration used. The correct formulation, route of administration, dosage, and interval between administrations should be selected according to methods known in the art and taking into account the particularity of individual conditions.

The dose and interval can be individually adjusted to provide a plasma level of the active part sufficient to achieve the desired effect; that is, the minimal effective concentration (MEC). The MEC of each compound will be different, but can be estimated, for example, from in vitro data and animal experiments. The dose necessary to obtain MEC will depend on individual characteristics and route of administration. In the case of local administration or selective uptake, the effective local concentration of the drug may not be related to plasma concentration.

The amount of the medicament or composition administered can be determined by various factors, including the sex, age and weight of the individual to be treated, the severity of the disease, the method of administration, and the judgment of the prescribing physician.

When needed, the composition of the present invention can be provided by a packaging or dispensing device containing one or more unit dosage forms (containing the active ingredient). For example, the packaging or device may include metal or plastic foil (such as foam packaging) or glass and rubber stoppers. The packaging or dispensing device may be accompanied by instructions for medicines. It is also possible to prepare a composition containing the compound of the present invention formulated in a compatible pharmaceutical carrier, which is placed in an appropriate container and labeled for the treatment of a specified condition.

The compounds of the present invention are suitable for the prevention and/or treatment of various diseases and conditions related to diseases, especially those characterized by increased plasma aldosterone concentration or changes in plasma aldosterone concentration relative to plasma renin concentration, or related illnesses related to these changes. Examples of that may be mentioned are: spontaneous primary aldosteronism, hyperaldosteronism associated with adrenal hyperplasia, adrenal adenoma and/or adrenal cancer, hyperaldosteronism associated with cirrhosis, hyperaldosteronism associated with heart failure, and (relative) hyperaldosteronism associated with essential hypertension, *etc.*

Due to its mechanism of action, the compounds of the present invention are also suitable for preventing sudden cardiac death in patients with an increased risk of death from sudden cardiac death. These patients are especially those suffering from one of the following conditions: primary and secondary hypertension, hypertensive heart disease with or without congestive heart failure, refractory hypertension, acute and chronic heart failure failure, coronary heart disease, stable and unstable angina pectoris, myocardial ischemia, myocardial infarction, dilated cardiomyopathy, congenital primary cardiomyopathy (such as Bmgada syndrome), cardiomyopathy caused by Chagas disease, shock, arteriosclerosis, atrial and ventricular arrhythmia, transient and ischemic attacks, stroke, inflammatory cardiovascular disorders, peripheral and cardiovascular disorders, peripheral blood flow disorders, arterial occlusive diseases such as intermittent claudication, asymptomatic left ventricle dysfunction, myocarditis, hypertrophic changes in the heart, pulmonary hypertension, coronary and peripheral arterial spasms, thrombosis, thromboembolic disorders and vasculitis.

The compounds of the present invention can additionally be used to prevent and/or treat the formation of edema, such as pulmonary edema, nephrogenic edema or swelling lungs associated with heart failure, and for example restenosis after thrombolytic therapy, percutaneous transluminal angioplasty (PTA) and percutaneous transluminal coronary angioplasty (PTCA), heart transplantation, and by-pass operation.

The compounds of the present invention are also suitable for use as potassium-sparing diuretics and for the treatment of electrolyte disorders such as hypercalcemia, hypernatremia or hypokalemia.

The compounds of the present invention are also suitable for the treatment of renal diseases such as acute and chronic renal failure, hypertensive nephropathy, arteriosclerotic nephritis (chronic and interstitial), nephrosclerosis, chronic renal failure and cystic nephropathy, for the prevention of kidney damage (For example, kidney damage caused by immunosuppressants related to organ transplantation (for example, cyclosporin A)) and for kidney cancer.

The compounds of the present invention can additionally be used to prevent and/or treat diabetes and diabetic sequelae, such as neuropathy and nephropathy.

The compound of the present invention can be further used for the prevention and/or treatment of microalbuminuria, such as caused by diabetes or hypertension, and proteinuria.

The compounds of the present invention are also suitable for the prevention and/or treatment of conditions related to an increase of plasma glucocorticoid concentration or a local increase of glucocorticoid concentration in tissues (for example, the heart). Examples that can be mentioned are: adrenal dysfunction (Cushing's syndrome) that leads to excessive production of glucocorticoids, adrenal cortical tumors that lead to excessive production of glucocorticoids, and pituitary tumors, which autonomously produce ACTH (adrenocorticotrophic hormone) leading to adrenal hyperplasia and Cushing's disease.

The compounds of the present invention can additionally be used to prevent and/or treat obesity, metabolic syndrome and obstructive sleep apnea.

The compounds of the present invention can be further used to prevent and/or treat inflammatory disorders caused by viruses, spirochetes, fungi, bacteria or mycobacteria, and inflammatory disorders of unknown etiology, such as polyarthritis, lupus erythematosus, joints peripheral inflammation or polyarteritis, dermatomyositis, scleroderma and sarcoidosis.

The compounds of the present invention can be further used to treat central nervous system disorders, such as depression, anxiety, and chronic pain, especially migraine, and neurodegenerative disorders, such as Alzheimer's disease and Parkinson's syndrome.

The compounds of the present invention are also suitable for the prevention and/or treatment of vascular injury, for example, percutaneous transluminal coronary angioplasty (PTCA), stent implantation, coronary angioscopy, vascular injury resulting from reocclusion or restenosis after bypass surgery, and endothelial dysfunction, Raynaud's disease, thromboangiitis obliterans (Buerger's syndrome) and tinnitus syndrome.

The compound of the present invention can be used alone, or if necessary, can be used in combination with other active ingredients. The present invention further relates to a medicament comprising at least one compound of the present invention and one or more other active ingredients (especially for the treatment and/or prevention of the aforementioned conditions), especially a drug combination for the treatment and/or prevention of the diseases mentioned in the present invention. Suitable active ingredients for the combination include, but are not limited to: active ingredients that lower blood pressure, such as and preferably selected from calcium antagonists, angiotensin II receptor antagonists, ACE inhibitors, endothelin antagonists, renin inhibitors, α-receptor blockers, β-receptor blockers and Rho kinase inhibitors; diuretics, especially loop diuretics, and thiazides and thiazide diuretics; agents having antithrombotic effects, for example and preferably selected from platelet aggregation inhibitors, anticoagulants or fibrinolytic substances; active ingredients that alter lipid metabolism, such as and preferably selected from thyroid receptor agonists, cholesterol synthesis inhibitors such as and preferably HMG-Coenzyme A reductase inhibitor or squalene synthesis inhibitor, ACAT inhibitor, CETP inhibitor, bile acid reuptake inhibitor and lipoprotein (a) antagonist; organic nitrate and NO donor, such as sodium nitroprusside, nitroglycerin, isosorbide mononitrate, isosorbide dinitrate, molsidomine or SIN-1, and inhaled NO; compounds with positive inotropic effects, such as cardiac glycosides (digoxin), isoproterenol, epinephrine, norepinephrine, dopamine and dobutamine; componds that inhibit cyclic-guanosine phosphate (cGMP) and/or cyclic-adenosine phosphate (cAMP) decomposition, such as phosphodiesterase (PDE) 1, 2, 3, 4 and/or 5 inhibitors (such as sildenafil, vardenafil, tadalafil, amrinone and milrinone); natriuretic peptides, such as atrial natriuretic peptide, B-type natriuretic peptide or brain natriuretic peptide, C-type natriuretic peptide (CNP) and urodilatin; calcium sensitizers, such as and preferably levosimendan; NO-independent but heme-dependent guanylate cyclase stimulator, especially compounds described in WO 00/06568, WO00/06569, WO02/42301 and WO03/095451 (for example, Riociguat); NO- and heme-independent guanylate cyclase activators, especially compounds described in WO 01/19355, WO 01/19776, WO 01/19778, WO 02/070462 and WO 02/070510 compounds; human neutrophil elastase (HNE) inhibitors, such as sivelestone or DX-890 (Reltran); compounds that inhibit signal transduction cascades, such as tyrosine kinase inhibitors, especially sorafenib, imatinib, gefitinib, and erlotinib; and/or compounds that affect cardiac energy metabolism, such as etomoxir, dichloroacetate, ranolazine or trimetazidine.

The compound of the present invention can also be administered in combination with other active ingredients other than the above-mentioned active ingredients. For example, in a preferred embodiment of the present invention, the compound of the present invention can be adminstered in combination with diuretics such as furosemide, bumetanide, torsemide, bendrofluazide, chlorothiazide, hydrochlorothiazide, hydrofluoromethiazide, methyclothiazide, polythiazide, trichlorothiazide, chlorthalidone, indapamide, metolazone, quinethazone, acetazolamide, dichlorobenzenesulfonamide, methazolamide, glycerin, isosorbitol, mannitol, amiloride or triamterene.

### GENERAL SYNTHETIC PROCEDURES

In the present invention, if the chemical name of the compound doesn't match the corresponding structure, the compound is characterized by the corresponding structure.

Generally, the compounds disclosed herein may be prepared by methods described herein, wherein the substituents are as defined for Formula (I) above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, *e.g.,* by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Inc., Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Qingdao Tenglong Reagent Chemical Ltd., and Qingdao Ocean Chemical Factory.

Anhydrous THF, dioxane, toluene, and diethyl ether were obtained by refluxing the solvent with sodium. Anhydrous CH₂Cl₂ and CHCl₃ were obtained by refluxing the solvent with CaH₂. EtOAc, PE, *n*-hexane, *N,N*-dimethylacetamide and *N,N-*dimethylformamide were treated with anhydrous Na₂SO₄ prior use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Column chromatography was conducted using a silica gel column. Silica gel (300-400 mesh) was purchased from Qingdao Ocean Chemical Factory. ¹H NMR spectra were recorded by a Bruker Avance 400 MHz spectrometer or Bruker Avance III HD 600 spectrometer, using CDCl₃, DMSO-*d₆*, CD₃OD or acetone-*d₆* (reported in ppm) as solvent, and using TMS (0 ppm) or chloroform (7.25 ppm) as the reference standard. When peak multiplicities are reported, the following abbreviations are used: s (singlet), d (doublet), t (triplet), m (multiplet), q (quartet), br (broadened), dd (doublet of doublets), dt (doublet of triplets), dq (doublet of quartets), ddd (doublet of doublet of doublets), ddt (doublet of doublet of triplets), dddd (doublet of doublet of doublet of doublets). Coupling constants, when given, were reported in Hertz (Hz).

Low-resolution mass spectral (MS) data were determined by an Agilent 6320 Series LC-MS spectrometer equipped with a G1312A binary pump and a G1316A TCC (column was operated at 30 °C). G1329A autosampler and G1315B DAD detector were applied in the analysis, and an ESI source was used in the LC-MS spectrometer.

Low-resolution mass spectral (MS) data were determined by an Agilent 6120 Series LC-MS spectrometer equipped with a G1311A quaternary pump and a G1316A TCC (column was operated at 30 °C). G1329A autosampler and G1315D DAD detector were applied in the analysis, and an ESI source was used on the LC-MS spectrometer.

Both LC-MS spectrometers were equipped with an Agilent Zorbax SB-C18, 2.1 x 30 mm, 5 µm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were shown in Table 1:

**Table 1: the gradient condition of the mobile phase in Low-resolution mass spectrum analysis**

| Time (min) | A (CH₃CN, 0.1% HCOOH) | B (H₂O, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

The following abbreviations are used throughout the specification: DMSO-*d₆* Deuterated dimethyl sulfoxide; g gram; mg milligram; mol mole; mmol millimole; mL milliliter; µL microliter

The following synthetic schemes describe the steps for preparing the compounds disclosed herein. Wherein, unless otherwise state, R¹, R², R³, R⁴, R⁶, R⁷, R⁸ and R^{x} are as defined herein; L is a leaving group, such as Cl, Br, I, methylsulfonyl, *p*-toluenesulfonyl, and the like, R⁰ is unsubstituted or substituted C₁₋₆ alkyl (such as methyl, ethyl, *tert*-buyl, phenylmethyl (*i.e.,* benzyl), cyanoethyl, trimethylsilylethyl, and the like). Unless otherwise specified, the reaction steps in each reaction scheme of the present invention are all performed in a solvent inert to the reaction. The solvent inert to the reaction includes, but is not limited to, the solvents involved in the embodiments of the present invention or their substitutes.

### Schemes

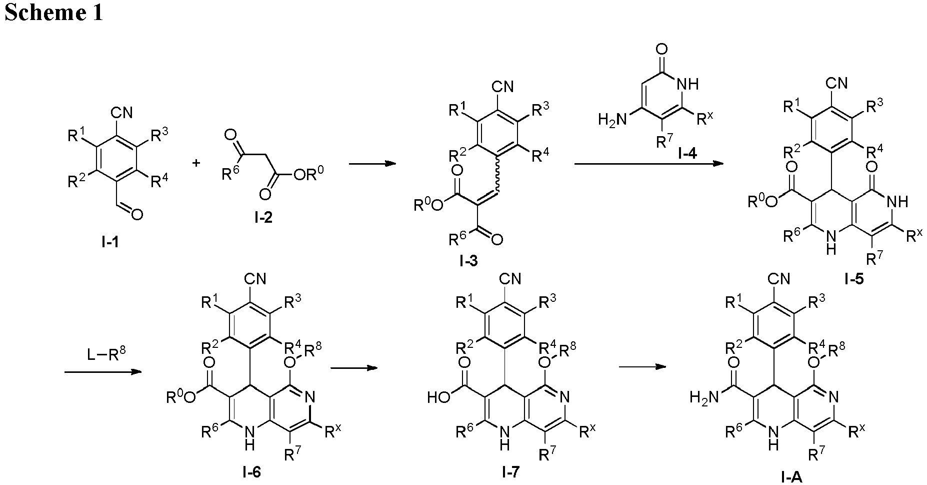

The compound represented by Formula **I-A** can be prepared according to the method described in **Scheme** 1. wherein, compound of Formula **1-1** can undergo aldol condensation with compound of Formula **1-2** under a suitable condition to give a compound of Formula **1-3,** then compound of Formula **1-3** can undergo cyclization with compound of Formula **1-4** under a suitable condition to give compound **1-5.** Compound of Formula **1-5** can react with compound L-R⁸ under an alkaline condition (such as in the presence of cesium carbonate, potassium carbonate, silver carbonate or sodium cyanide) to give compound of Formula **1-6.** Compound of Formula **1-6** can undergo deprotection (hydrolysis or palladium-carbon hydrogenation reduction, *etc*.) to give compound of Formula **1-7,** and then compound of Formula **1-7** can undergo condensation reaction to give compound of Formula **I-A.**

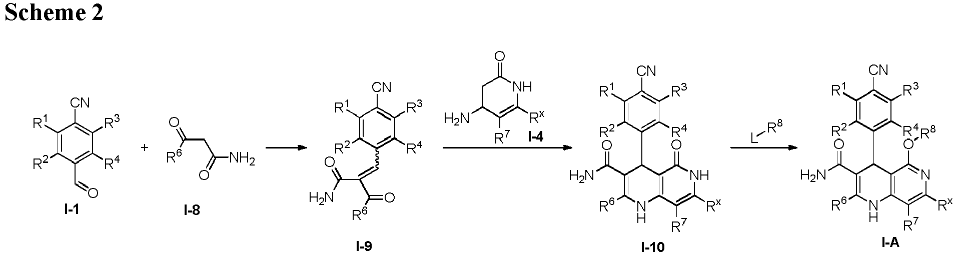

Compound of Formula **I-A** can be prepared according to the method described in **Scheme 2.** wherein, compound of Formula **1-1** can undergo aldol condensation with compound of Formula **1-8** to give a compound of Formula **1-9,** then compound of Formula **1-9** can undergo cyclization with compound of Formula **1-4** under a suitable condition to give compound of Formula **I-10.** Compound of Formula **I-10** can react with compound L-R⁸ under a alkaline condition (such as in the presence of cesium carbonate, potassium carbonate, silver carbonate or sodium cyanide) to give compound of Formula **I-A.**

The following examples disclosed herein are presented to further describe the invention. However, these examples should not be used to limit the scope of the invention.

### Examples

### Example 1 4-(4-Cyano-2-methoxyphenyl)-5-(cyclopropylmethoxy)-2,8-dimethyl-1,4-dihydro-1,6-naphthyridine-3-carboxamide

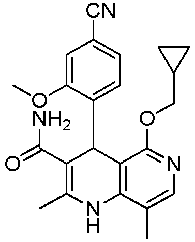

### Step 1) Benzyl 2-(4-cyano-2-methoxyphenylmethylene)-3-oxobutyrate

4-Cyano-2-methoxybenzaldehyde (10.0 g, 62.1 mmol), benzyl acetoacetate (11.9 g, 61.9 mmol), piperidine (1.06 g, 12.4 mmol) and acetic acid (0.74 g, 12 mmol) were added into dichloromethane (80 mL). The reaction flask was equipped with an oil-water separator, and the mixture in the reaction flask was refluxed over night. The mixture was cooled to room temperature, and the solvent was distilled off. The residue was purified by silica gel column chromatography (petroleum ether/ethyl acetate (v/v) = 20/1) to give a light yellow solid (12.9 g, 62.0%).

### Step 2) Benzyl 4-(4-cyano-2-methoxyphenyl)-2,8-dimethyl-5-oxo-1,4,5,6-tetrahydro-1,6 -naphthyridine-3-carboxylate

Benzyl 2-(4-cyano-2-methoxyphenylmethylene)-3-oxobutyrate (12.9 g, 38.5 mmol) and 4-amino-5-methyl-2-hydroxypyridine (5.25 g, 42.3 mmol) were added into isopropanol (80 mL). The mixture was refluxed overnight. The mixture was cooled to room temperature, and filtered by suction, and the filter cake was washed with isopropanol (80 mL), dried *in vacuo* to give a light yellow solid (11.3 g, 66.5%).
MS (ESI, pos.ion) m/z: 442.2 (M+1).

### Step 3) Benzyl 4-(4-cyano-2-methoxyphenyl)-5-(cyclopropylmethoxy)-2,8-dimethyl -1,4-dihydro-1,6-naphthyridine-3-carboxylate

Benzyl 4-(4-cyano-2-methoxyphenyl)-2,8-dimethyl-5-oxo-1,4,5,6-tetrahydro-1,6-naphthyridine-3-carboxylate (5.0 g, 11 mmol) and cesium carbonate (5.50 g, 16.9 mmol) were added into *N,N*-dimethylformamide (30 mL), then to the resulting mixture was added bromomethylcyclopropane (1.60 mL, 16.5 mmol). After addition, the mixture reacted overnight. The mixture was poured into water, extracted with ethyl acetate (80 mL × 2). The combined organic phases were washed with water (80 mL × 2) and saturated brine (80 mL), dried over anhydrous sodium sulfate. The mixture was cooled to room temperature, and the solvent was distilled off. The crude product was purified by silica gel column chromatography (petroleum ether / ethyl acetate (v/v) = 3/1) to give a light yellow solid (0.96 g, 17%).

### Step 4) 4-(4-Cyano-2-methoxyphenyl)-5-(cyclopropylmethoxy)-2,8-dimethyl-1,4-dihydro -1,6-naphthyridine-3-carboxylic acid

Benzyl 4-(4-cyano-2-methoxyphenyl)-5-(cyclopropylmethoxy)-2,8-dimethyl-1,4-dihydro -1,6-naphthyridine-3-carboxylate (0.96 g, 1.9 mmol) and 10% Pd/C (0.10 g) were added into methanol (20 mL). The mixture was stirred in hydrogen atomosphere for 1 hour at room temperature. The mixture was filtered, and the filtrate was distilled to remove the solvent and a light yellow solid (0.66 g, 84%) was obtained.
MS (ESI, pos.ion) m/z: 406.2 (M+1).

### Step 5) 4-(4-Cyano-2-methoxyphenyl)-5-(cyclopropylmethoxy)-2,8-dimethyl-1,4-dihydro-1,6-naphthyridine-3-formamide

4-(4-Cyano-2-methoxyphenyl)-5-(cyclopropylmethoxy)-2,8-dimethyl-1,4-dihydro-1,6-naphthyridine-3-carboxylic acid (0.65 g, 1.6 mmol) and ammonium chloride (0.26 g, 4.9 mmol) were added into *N,N*-dimethylformamide (30 mL), then O-(7-azabenzotriazol-1-yl)-*N,N,N',N'*-tetramethyluronium hexafluorophosphate (0.79 g, 2.1 mmol) and triethylamine (0.67 mL, 4.8 mmol) were added into the resulting mixture at 0°C. After addition, the mixture was stirred at room temperature. To the mixture was added water to quench the mixture, and the resulting mixture was extracted with ethyl acetate (60 mL x 2). The combined organic phases were washed with water (80 mL) and saturated brine (80 mL), dried over anhydrous sodium sulfate. The mixture was filtered, and the filtrate was concentrated by rotary evaporator to dry, and the residue was purified by silica gel chromatography (dichloromethane / MeOH (v/v) = 100/1) to give a light yellow solid (0.36 g, 56%).
MS (ESI, pos. ion) m/z: 405.3 (M+1); ¹H NMR (400 MHz, CDCl₃) δ (ppm) 7.67 (s, 1H), 7.33 (d, *J* = 7.9 Hz, 1H), 7.18 (dd, *J =* 7.9, 1.2 Hz, 1H), 7.10 (d, *J =* 0.8 Hz, 1H), 5.79 (s, 1H), 5.50 (s, 1H), 5.32 (s, 2H), 4.09 - 3.94 (m, 4H), 3.89 (dd, *J=* 11.0, 7.3 Hz, 1H), 2.52 (s, 3H), 2.18 (s, 3H), 1.10 - 1.00 (m, 1H), 0.56 - 0.40 (m, 2H), 0.25 - 0.09 (m, 2H).

### Example 2 4-(4-Cyano-2-methoxyphenyl)-5-(3,3-difluorocyclobutyl)methoxy)-2,8 -dimethyl-1,4-dihydro-1,6-naphthyridine-3-carboxamide

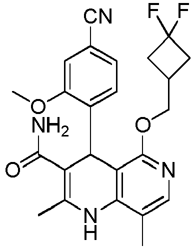

### Step 1) (3,3-difluorocyclobutyl)methyl methanesulfonate

To a reaction flask were added (3,3-difluorocyclobutyl)methanol (500 mg, 4.09 mmol), triethylamine (1.14 mL, 8.20 mmol) and dichloromethane (20 mL). To the mixture was slowly added dropwise methylsulfonyl chloride (0.38 mL, 4.8 mmol). After addition, the resulting mixture was stirred for 14 hours. The reaction was quenched with water (40 mL) . The resulting mixture was extracted with dichloromethane (30 mL × 2). The combined organic layers were dried over anhydrous sodium sulfate, filtered, and the solvent was evaporated under reduced pressure to give light yellow liquid (819 mg, 99.91%).
¹H NMR (400 MHz, CDCl₃) δ (ppm) 4.28 (d, *J* = 6.5 Hz, 2H), 3.06 (s, 3H), 2.81 - 2.68 (m, 2H), 2.67 - 2.54 (m, 1H), 2.51 - 2.34 (m, 2H).

### Step 2) Benzyl 4-(4-cyano-2-methoxyphenyl)-5-((3,3-difluorocyclobutyl)methoxy) -2,8-dimethyl-1,4-dihydro-1,6-naphthyridine-3-carboxylate

To a reaction flask were added Benzyl 4-(4-cyano-2-methoxyphenyl) -2,8-dimethyl-5-oxo-1,4,5,6-dihydro-1,6-naphthyridine-3-formamide (500 mg, 1.13 mmol), cesium carbonate (738 mg, 2.26 mmol) and *N,N*-dimethylformamide (20 mL). Then to the mixture was added dropwise (3,3-difluorocyclobutyl)methyl methanesulfonate (340 mg, 1.70 mmol). After addition, the mixture was heated to 60°C and reacted for 11 hours. The mixture was cooled to room temperature, quenched with water (20 mL) and extracted with ethyl acetate (40 mL × 2). The combined organic layers were washed with saturated saline (20 mL × 2), dried over anhydrous sodium sulfate, filtered, and distilled to remove the solvent, and the crude product was purified by silica gel chromatography (petroleum ether/ethyl acetate (v/v) = 2/1) to give a light yellow solid (220 mg, 35.60%).
MS (ESI, pos.ion) m/z: 546.2 (M+1).

### Step 3) 4-(4-Cyano-2-methoxyphenyl)-5-((3,3-difluorocyclobutyl)methoxy)-2,8-dimethyl -1,4-dihydro-1,6-naphthyridine-3-carboxylic acid

To a reaction flask were added benzyl 4-(4-cyano-2-methoxyphenyl) -5-((3,3-difluorocyclobutyl)methoxy)-2,8-dimethyl-1,4-dihydro-1,6-naphthyridine-3-carboxylate (220 mg, 0.40 mmol) and tetrahydrofuran (10 mL). To the mixture was added 10% Pd/C (21 mg). The reaction mixture was stirred at room temperature for 6.5 hours. The mixture was filtered, and distilled to remove the solvent. The crude product was purified by silica gel chromatography (petroleum ether/EtOAc (v/v) = 1/1) to give a light yellow solid (120 mg, 65.35%).

### Step 4) 4-(4-Cyano-2-methoxyphenyl)-5-((3,3-difluorocyclobutyl)methoxy)-2,8-dimethyl -1,4-dihydro-1,6-naphthyridine-3-carboxamide

To a reaction flask were added 4-(4-cyano-2-methoxyphenyl)-5-((3,3-difluorocyclobutyl)methoxy)-2,8-dimethyl-1,4-dihydro-1,6-naphthyridine-3-carboxylic acid (120 mg, 0.256 mmol), ammonium chloride (69 mg, 1.29 mmol), O-(7-azabenzotriazol-1-yl)-*N,N,N',N'*-tetramethyluronium hexafluorophosphate (202 mg, 0.51 mmol), *N,N*-dimethylformamide (10 mL) and triethylamine (0.22 mL, 1.60 mmol). The resulting mixture in the reaction flask were heated to 50°C and stirred for 15 hours. The mixture was cooled to room temperature, adjusted with saturated aqueous sodium bicarbonate to pH 8, then the resulting mixture was extracted with ethyl acetate (20 mL × 2). The combined organic phases were washed with saturated aqueous sodium bicarbonate (5 mL) and saturated brine (10 mL), and dried over anhydrous sodium sulfate. Then the mixture was filtered, and the filtrate was distilled under reduced pressure to remove the solvent. The crude product was purified by silica gel column chromatography (dichloromethane/methanol (v/v) = 100/1-50/1), then triturated with *n*-hexane/ethyl acetate (20 mL/2 mL)) for 2 hours, filtered. The filtrate was filtered and the filter cake was dried to give a white solid (50 mg, 42.60%).
MS (ESI, pos.ion) m/z: 455.7 (M+1);
¹H NMR (400 MHz, DMSO-*d₆*) δ (ppm) 7.70 (s, 1H), 7.57 (s, 1H), 7.39 (s, 1H), 7.29 (d, *J* = 9.0 Hz, 1H), 7.12 (d, *J* = 7.9 Hz, 1H), 6.84-6.71 (m, 2H), 5.37 (s, 1H), 4.13-4.02 (m, 2H), 3.80 (s, 3H), 2.45-2.40 (m, 2H), 2.35 - 2.16 (m, 2H), 2.14 (d, *J=* 4.4 Hz, 6H).

### Example 3 4-(4-Cyano-2-methoxyphenyl)-5-(cyclopentylmethoxy)-2,8-dimethyl-1,4 -dihydro-1,6-naphthyridine-3-carboxamide

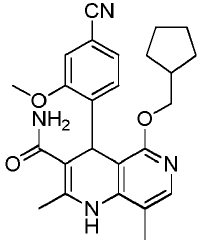

### Step 1) Cyclopentylmethyl methanesulfonate

To a reaction flask were added cyclopentylmethanol (500 mg, 4.99 mmol), triethylamine (1.39 mL, 10.0 mmol) and dichloromethane (20 mL). To the mixture was added dropwise methylsulfonyl chloride (0.47 mL, 6.0 mmol). After addition, the resulting mixture was stirred for 43 hours at room temperature. The reaction was quenched with water (40 mL) . The resulting mixture was extracted with DCM (30 mL × 2). The combined organic layers were dried over anhydrous sodium sulfate, filtered, and the filtrate was distilled under reduced pressure to remove the solvent and light yellow liquid (830 mg, 93.28%) was obtained.
¹H NMR (400 MHz, CDCl₃) δ (ppm) 4.12 (d, *J* = 7.1 Hz, 2H), 3.02 (s, 3H), 2.32 (dt, *J* = 15.0, 7.5 Hz, 1H), 1.91 - 1.75 (m, 2H), 1.62 (dt, *J=* 8.0, 6.2 Hz, 4H), 1.31 (dt, *J=* 13.7, 6.9 Hz, 2H).

### Step 2) Benzyl 4-(4-cyano-2-methoxyphenyl)-5-(cyclopropylmethoxy)-2,8-dimethyl -1,4-dihydro-1,6-naphthyridine-3-carboxylate

To a reaction flask were added benzyl 4-(4-cyano-2-methoxyphenyl) -2,8-dimethyl-5-oxo-1,4,5,6-dihydro-1,6-naphthyridine-3-carboxylate (500 mg, 1.13 mmol), cesium carbonate (738 mg, 2.26 mmol) and *N*,*N*-dimethylformamide (20 mL). Then to the mixture was added cyclopentylmethyl methanesulfonate (302 mg, 1.69 mmol). After addition, the mixture was heated to 60°C and reacted for 17 hours. The mixture was cooled to room temperature, quenched with water (20 mL) and extracted with ethyl acetate (40 mL × 2). The combined organic phases were washed with saturated saline (20 mL × 2), dried over anhydrous sodium sulfate, filtered, and concentrated *in vacuo,* filtered, and distilled to remove the solvent, and the crude product was purified by silica gel column chromatography (petroleum ether/ethyl acetate (v/v) = 2/1) to give a light yellow solid (160 mg, 26.98%).

### Step 3) 4-(4-Cyano-2-methoxyphenyl)-5-(cyclopropylmethoxy)-2,8-dimethyl-1,4-dihydro -1,6-naphthyridine-3-carboxylic acid

To a reaction flask were added benzyl 4-(4-cyano-2-methoxyphenyl) -5-(cyclopentylmethoxy)-2, 8-dimethyl-1,4-dihydro-1,6-naphthyridine-3 -carboxylate (160 mg, 0.31 mmol) and tetrahydrofuran (10 mL). To the mixture was added 10% Pd/C (32 mg). The reaction mixture was stirred at room temperature for 3 hours, filtered, and the filtrate was distilled to remove the solvent, and the crude product was purified by silica gel column chromatography (petroleum ether/ethyl acetate (v/v) = 1/1) to give a light yellow solid (65 mg, 49.07%).

### Step 4) 4-(4-Cyano-2-methoxyphenyl)-5-(cyclopentylmethoxy)-2,8-dimethyl-1,4-dihydro -1,6-naphthyridine-3-carboxamide

To a reaction flask were added 4-(4-cyano-2-methoxyphenyl)-5-(cyclopentylmethoxy)-2,8-dimethyl-1,4-dihydro-1,6-naphthyridine-3-carboxylic acid (65 mg, 0.15 mmol), ammonium chloride (40 mg, 0.75 mmol), O-(7-azabenzotriazol-1-yl)-*N,N,N',N'*-tetramethyluronium hexafluorophosphate (117 mg, 0.30 mmol), *N,N*-dimethylformamide (10 mL) and triethylamine (0.13 mL, 0.93 mmol). The resulting mixture was stirred for 15 hours at room temperature. The mixture was adjusted with saturated aqueous sodium bicarbonate to pH 8, extracted with ethyl acetate (20 mL × 2). The combined organic layers were washed with saturated aqueous sodium bicarbonate (5 mL) and saturated brine (10 mL), dried over anhydrous sodium sulfate. Then filtered, and the filtrate was distilled under reduced pressure to remove the solvent. The crude product was purified by silica gel column chromatography (dichloromethane/methanol (v/v) = 100/1-50/1), then triturated with (*n*-hexane/ethyl acetate (20 mL/2mL)) for 2 hours, filtered. The filtrate was filtered and the filter cake was dried to give a white solid (58 mg, 89.44%).
MS (ESI, pos.ion) m/z: 433.2 (M+1);
¹H NMR (400 MHz, DMSO-*d₆*) δ (ppm) 7.67 (s, 1H), 7.55 (s, 1H), 7.38 (s, 1H), 7.28 (d, *J* = 7.8 Hz, 1H), 7.11 (d, *J* = 7.8 Hz, 1H), 6.82-6.70 (m, 2H), 5.38 (s, 1H), 3.97-3.93 (m, 1H), 3.87 - 3.81 (m, 1H), 3.80 (s, 3H), 2.15 (s, 3H), 2.13 (s, 3H), 2.07 - 1.98 (m, 1H), 1.50-1.45 (m, 6H), 1.11 - 0.91 (m, 2H).

### Example 4 4-(4-Cyano-2-methoxyphenyl)-2,8-dimethyl-5-(oxetan-3-yloxy)-1,4-dihydro -1,6-naphthyridine-3-carboxamide

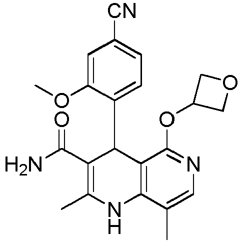

### Step 1) Benzyl 4-(4-cyano-2-methoxyphenyl)-2,8-dimethyl-5-(oxetan-3-yloxy)-1,4-dihydro -1,6-naphthyridine-3-carboxylate

To a 50 mL two-neck flask were added benzyl 4-(4-cyano -2-methoxyphenyl)-2,8-dimethyl-5-oxo-1,4,5,6-tetrahydro-1,6-naphthyridine-3-carboxylate (3.0 g, 6.8 mmol), cesium carbonate (4.9 g, 15 mmol) and *N,N*-dimethylformamide (30 mL). Then to the mixture was added 3-iodooxetane (1.9 g, 10 mmol). After addition, the mixture was stirred at room temperature overnight. To the mixture was added water (30 mL) to quench the mixture, and the resulting mixture was extracted with ethyl acetate (20 mL × 2). The combined organic phases were washed with water (30 mL) and saturated brine (30 mL), dried over anhydrous sodium sulfate. The mixture was filtered, and the filtrate was concentrated by rotary evaporator to dry, and the residue was purified by silica gel column chromatography (dichloromethane/methanol (v/v) = 80/1) to give a light yellow solid (0.31 g, 9.2%).
MS (ESI, pos.ion) m/z: 498.2 (M+1).

### Step 2) 4-(4-Cyano-2-methoxyphenyl)-2,8-dimethyl-5-(oxetan-3-yloxy)-1,4-dihydro-1,6 -naphthyridine-3-carboxylic acid

To a 50 mL single-neck flask were added benzyl 4-(4-cyano-2-methoxyphenyl)-2,8-dimethyl-5-(oxetan-3-yloxy)-1,4-dihydro-1,6-naphthyridine-3 -carboxylate (0.28 g, 0.56 mmol) and methanol (12 mL). To the mixture was added 10% Pd/C (0.10 g). The reaction mixture was stirred at room temperature in hydrogen atomosphere for 0.5 hours. The mixture was fitered, and the filtrate was washed with ethyl acetate (20 mL), evaporated by rotary evaporator to dry and give a light yellow solid (0.19 g, 83%).
MS (ESI, pos.ion) m/z: 408.3 (M+1).

### Step 3) 4-(4-Cyano-2-methoxyphenyl)-2,8-dimethyl-5-(oxetan-3-yloxy)-1,4-dihydro-1,6 - naphthyridine-3-carboxamide

To a 50 mL single-neck flask were added 4-(4-cyano-2-methoxyphenyl)-2,8-dimethyl-5-(oxetan-3-yloxy)-1,4-dihydro-1,6-naphthyridine-3-carboxylic acid (0.090 g, 0.22 mmol), ammonium chloride (0.024 g, 0.45 mmol) and *N,N*-dimethylformamide (6 mL), then O-(7-azabenzotriazol-1-yl)-*N,N,N*'*,N*'-tetramethyluronium hexafluorophosphate (0.13 g, 0.34 mmol) and *N,N*-diisopropylethylamine (0.11 mL, 0.67 mmol) were added into the resulting mixture at 0°C. The mixture was stirred at room temperature overnight. To the mixture was added water (20 mL), and the resulting mixture was extracted with ethyl acetate (30 mL × 2). The combined organic phases were washed with water (30 mL) and saturated brine (30 mL), dried over anhydrous sodium sulfate. The mixture was filtered, and the filtrate was concentrated by rotary evaporator to dry, and the residue was purified by silica gel chromatography (dichloromethane/MeOH (v/v) = 50/1) to give a white solid (0.037 g, 41%).
MS (ESI, pos.ion) m/z: 407.2 (M+1);
¹H NMR (400 MHz, DMSO-*d₆*) δ (ppm) 7.76 (s, 1H), 7.51 (s, 1H), 7.40 (s, 1H), 7.30 (d, *J* = 7.8 Hz, 1H), 7.19 (d, *J* = 7.9 Hz, 1H), 6.78 (d, *J* = 44.1 Hz, 2H), 5.47 (s, 1H), 5.36 - 5.28 (m, 1H), 4.77 (m, 1H), 4.65 (m, 1H), 4.44 - 4.35 (m, 1H), 4.15 - 4.05 (m, 1H), 3.84 (s, 3H), 2.19 (s, 3H), 2.12 (s, 3H).

### Example 5 4-(4-cyano-2-methoxyphenyl)-5-(cyclobutylmethoxy)-2,8-dimethyl-1,4-dihydro-1,6-naphthyridine-3-carboxamide

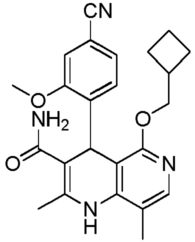

### Step 1) Benzyl 4-(4-cyano-2-methoxyphenyl)-5-(cyclobutylmethoxy)-2,8-dimethyl-1,4-dihydro-1,6-naphthyridine-3-carboxylate

Benzyl 4-(4-cyano-2-methoxyphenyl)-2,8-dimethyl-5-oxo-1,4,5,6-tetrahydro-1,6-naphthyridine-3-carboxylate (600 mg, 1.36 mmol), cesium carbonate (885 mg, 2.72 mmol) and bromomethylcyclobutane (0.3 g, 2 mmol) were dissolved in *N,N*-dimethylformamide (30 mL). The mixture was stirred at 60 °C overnight. The mixture was cooled to room temperature, diluted with water (50 mL). The resulting mixture was extracted with ethyl acetate (50 mL × 2), and the organic layers were combined, washed with saturated brine (30 mL × 2). The organic layers were collected, evaporated under reduced pressure to remove the solvent, and the residue was purified by silica gel column chromatography (petroleum ether/ethyl acetate (v/v) = 1/1) to give light yellow oil (320 mg, 46.21%).
MS (ESI, pos.ion) m/z: 510.1 (M+1).

### Step 2) 4-(4-Cyano-2-methoxyphenyl)-5-(cyclobutylmethoxy)-2,8-dimethyl-1,4-dihydro -1,6-naphthyridine-3-carboxylic acid

Benzyl 4-(4-cyano-2-methoxyphenyl)-5-(cyclobutylmethoxy)-2,8-dimethyl-1,4-dihydro-1,6-naphthyridine-3-carboxylate (320 mg, 0.63 mmol) and 10% Pd/C (65 mg, 0.06 mmol) were added into methanol (20 mL). The air in the reaction flask was replaced with hydrogen, and the mixture was stirred at room temperature for 5 minutes. The reaction mixture was filtered through a celite pad, and the filtrate was evaporated under reduced pressure to remove the solvent. The residue was purified by silica gel column chromatography (petroleum ether/ethyl acetate (v/v) = 1/1) to give white and transparent oil (120 mg, 45.55%).
MS (ESI, pos.ion) m/z: 420.2 (M+1).

### Step 3) 4-(4-Cyano-2-methoxyphenyl)-5-(cyclobutylmethoxy)-2,8-dimethyl-1,4-dihydro -1,6-naphthyridine-3-carboxamide

Ammonium chloride (46 mg, 0.86 mmol), 4-(4-cyano-2-methoxyphenyl)-5-(cyclobutylmethoxy)-2,8-dimethyl-1,4-dihydro-1,6-naphthyridine-3-carboxylic acid (120 mg, 0.29 mmol), O-(7-azabenzotriazol-1-yl)-*N*,*N*,*N*',*N*'-tetramethyluronium hexafluorophosphate (220 mg, 0.58 mmol) and *N,N*-diisopropylethylamine (0.5 mL, 3 mmol) were dissolved in *N,N*-dimethylformamide (30 mL). The resulting mixture was stirred overnight at room temperature. The mixture was diluted with water (50 mL), and extracted with ethyl acetate (50 mL × 2), and the organic layers were combined, washed with saturated brine (30 mL × 2). The organic layers were collected, evaporated under reduced pressure to remove the solvent, and the residue was purified by silica gel chromatography (dichloromethane/methanol(v/v) = 15/1) to give a white solid (40 mg, 33.41%).
MS (ESI, pos.ion) m/z: 419.1 (M+1);
¹H NMR (400 MHz, CDCl₃) δ (ppm) 7.69 (s, 1H), 7.30 (d, *J* = 8.1 Hz, 1H), 7.19 - 7.15 (m, 1H), 7.09 (s, 1H), 5.81 (s, 1H), 5.43 (s, 1H), 4.16 - 4.10 (m, 2H), 3.99 (s, 3H), 2.60 - 2.52 (m, 1H), 2.50 (s, 3H), 2.19 (s, 3H), 2.02- 1.85 (m, 4H), 1.70 (d, *J* = 10.2 Hz, 2H).

### Example 6 4-(4-Cyano-2-methoxyphenyl)-2,8-dimethyl-5-(oxetan-3-ylmethoxy)-1,4-dihydro-1,6-naphthyridine-3-carboxamide

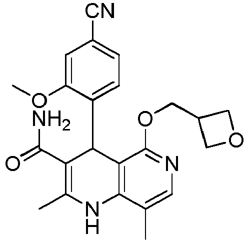

### Step 1) 2-(4-Cyano-2-methoxyphenylmethylene)-3-oxobutanamide

4-Cyano-2-methoxybenzaldehyde (38.6 g, 240 mmol), 3-oxobutanamide (22.0 g, 218 mmol), piperidine (2.0 mL, 22 mmol) and acetic acid (1.25 mL, 21.8 mmol) were added into dichloromethane (150 mL). The reaction flask was equipped with an oil-water separator, and the mixture in the reaction flask was refluxed overnight. The mixture was cooled to room temperature, filtered by suction, and the filter cake was washed with dichloromethane (50 mL), water (100 mL × 2) and ethanol (100 mL), dried *in vacuo* to give a light yellow solid (41.3 g, 77.7%).
MS (ESI, pos.ion) m/z: 245.1 (M+1).

### Step 2) 4-(4-Cyano-2-methoxyphenyl)-2,8-dimethyl-5-oxo-1,4,5,6-tetrahydro-1,6-naphthyridine-3-formamide

2-(4-Cyano-2-methoxyphenylmethylene)-3-oxobutanamide (10.8 g, 44.2 mmol), acetic acid (2.3 mL, 40 mmol) and 4-amino-5-methyl-2-hydroxypyridine (5.00 g, 40.3 mmol) were added into isopropanol (100 mL). The mixture was stirred at 120°C in a sealed tub for 48 hours. The mixture was cooled to room temperature, and filtered to give a light yellow solid (6.31 g, 44.7%).

### Step 3) 4-(4-Cyano-2-methoxyphenyl)-2,8-dimethyl-5-(oxetan-3-ylmethoxy)-1,4-dihydro-1,6-naphthyridine-3-carboxamide

4-(4-Cyano-2-methoxyphenyl)-2,8-dimethyl-5-oxo-1,4,5,6-tetrahydro-1,6-naphthyridine-3-f ormamide (300 mg, 0.86 mmol), cesium carbonate (557 mg, 1.71 mmol) and 3-(bromomethyl)oxetane (193 mg, 1.28 mmol) were dissolved in *N,N*-dimethylformamide (20 mL). The mixture was stirred at 60 °C overnight. The mixture was cooled to room temperature, diluted with water (50 mL) and extracted with ethyl acetate (50 mL × 2). The combined organic phases were washed with saturated saline (30 mL × 2), dried over anhydrous sodium sulfate, filtered, and concentrated *in vacuo.* The mixture was filtered, and the filtrate was concentrated by rotary evaporator to dry, and the residue was purified by silica gel column chromatography ( dichloromethane/methanol(v/v) = 20/1) to give a light yellow solid (5 mg, 1.39 %).
MS (ESI, pos.ion) m/z: 421.6 (M+1);
¹H NMR (400 MHz, CDCl₃) δ (ppm) 7.70 (s, 1H), 7.31 (s, 1H), 7.21 - 7.16 (m, 1H), 7.10 (s, 1H), 5.81 (s, 1H), 5.43 (s, 1H), 4.77 - 4.68 (m, 2H), 4.43 - 4.34 (m, 4H), 3.99 (s, 3H), 2.50 (s, 3H), 2.20 (s, 3H).

### Example 7 4-(4-Cyano-2-methoxyphenyl)-5-(cyclobutyloxy)-2,8-dimethyl-1,4-dihydro -1,6-naphthyridine-3-carboxamide

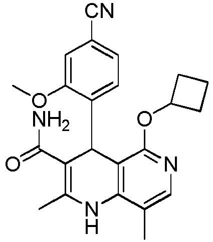

### Step 1) Benzyl 4-(4-cyano-2-methoxyphenyl)-5-(cyclobutyloxy)-2,8-dimethyl-1,4 -dihydro-1,6-naphthyridine-3-carboxylate

Benzyl 4-(4-cyano-2-methoxyphenyl)-2,8-dimethyl-5-oxo-1,4,5,6-tetrahydro-1,6-naphthyridine-3-carboxylate (500 mg, 1.13 mmol), cesium carbonate (740 mg, 2.27 mmol) and bromocyclobutane (0.2 mL, 2 mmol) were dissolved in *N,N*-dimethylformamide (20 mL). The mixture was stirred at 65 °C overnight. The mixture was cooled to room temperature, diluted with water (50 mL) and extracted with ethyl acetate (50 mL × 2). The combined organic phases were washed with saturated saline (30 mL × 2), dried over anhydrous sodium sulfate, filtered, and the filtrate was distilled *in vacuo* to remove the solvent, and the crude product was purified by silica gel column chromatography (petroleum ether/ethyl acetate (v/v) = 1/1) to give a light yellow solid (230 mg, 40.98%).
MS (ESI, pos.ion) m/z: 496.2 (M+1).

### Step 2) 4-(4-Cyano-2-methoxyphenyl)-5-(cyclobutyloxy)-2,8-dimethyl-1,4-dihydro -1,6-naphthyridine-3-carboxylic acid

Benzyl 4-(4-cyano-2-methoxyphenyl)-5-(cyclobutyloxy)-2,8-dimethyl-1,4-dihydro-1,6 -naphthyridine-3-carboxylate (230 mg, 0.46 mmol) and 10% Pd/C (40 mg) were added into tetrahydrofuran (20 mL). The mixture was stirred in hydrogen atomosphere for 6 hour at room temperature. The reaction mixture was filtered through a celite pad, and the filtrate was evaporated under reduced pressure to remove the solvent. The residue was purified by silica gel column chromatography (petroleum ether/ethyl acetate (v/v) = 1/2) to give a white solid (150 mg, 79.73%).

### Step 3) 4-(4-Cyano-2-methoxyphenyl)-5-(cyclobutyloxy)-2,8-dimethyl-1,4-dihydro -1,6-naphthyridine-3-carboxamide

Ammonium chloride (60 mg, 1.12 mmol), 4-(4-cyano-2-methoxyphenyl)-5-(cyclobutyloxy)-2,8-dimethyl-1,4-dihydro-1,6-naphthyridine-3-carboxylic acid (150 mg, 0.37 mmol), O-(7-azabenzotriazol-1-yl)-*N*,*N*,*N'*,*N'*-tetramethyluronium hexafluorophosphate (270 mg, 0.71 mmol) and *N*,*N-*diisopropylethylamine (0.5 mL, 3 mmol) were dissolved in *N*,*N*-dimethylformamide (20 mL). The resulting mixture was heated to 50 °C and stirred for 6 hours. The mixture was cooled to room temperature, diluted with water (50 mL) and extracted with ethyl acetate (50 mL × 2). The combined organic phases were washed with saturated saline (30 mL × 2), dried over anhydrous sodium sulfate. The mixture was filtered, and the filtrate was concentrated by rotary evaporator to dry, and the residue was purified by silica gel column chromatography (dichloromethane/methanol(v/v) = 25/1) to give a white solid (130 mg, 86.86%).
MS (ESI, pos.ion) m/z : 405.6 (M+1);
¹H NMR (400 MHz, CDCl₃) δ (ppm) 7.66 (s, 1H), 7.32 (d, *J*= 7.9 Hz, 1H), 7.18 (d, *J*= 7.9 Hz, 1H), 7.10 (s, 1H), 5.80 (s, 1H), 5.46 (s, 1H), 5.07 - 4.99 (m, 1H), 4.02 (s, 3H), 2.52 (s, 3H), 2.46 - 2.37 (m, 1H), 2.25 - 2.19 (m, 1H), 2.17 (s, 3H), 2.03 - 1.92 (m, 1H), 1.75 - 1.68 (m, 1H), 1.65 - 1.55 (m, 2H).

### Example 8 4-(4-Cyano-2-methoxyphenyl)-5-(cyclopentyloxy)-2,8-dimethyl-1,4-dihydro -1,6-naphthyridine-3-carboxamide

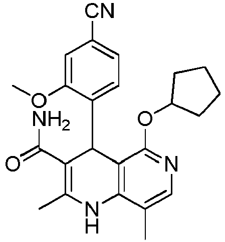

4-(4-Cyano-2-methoxyphenyl)-2,8-dimethyl-5-oxo-1,4,5,6-tetrahydro-1,6-naphthyridine -3-formamide (300 mg, 0.86 mmol), cesium carbonate (557 mg, 1.71 mmol) and bromocyclobutane (0.14 mL, 1.3 mmol were dissolved in *N*,*N*-dimethylformamide (20 mL). The mixture was stirred at 60 °C overnight. The mixture was cooled to room temperature, diluted with water (50 mL) and extracted with ethyl acetate (50 mL × 2). The combined organic phases were washed with saturated saline (30 mL × 2), dried over anhydrous sodium sulfate, filtered, and concentrated *in vacuo.* The residue was purified by silica gel column chromatography (dichloromethane/methanol(v/v) = 20/1) to give an off-white solid (20 mg, 5.58 %).
MS (ESI, pos.ion) m/z: 419.2 (M+1);
¹H NMR (400 MHz, CDCl₃) δ (ppm) 7.70 (s, 1H), 7.31 (d, *J*= 8.1 Hz, 1H), 7.17 (d, *J*= 7.8 Hz, 1H), 7.08 (s, 1H), 5.79 (s, 1H), 5.41 (s, 1H), 3.97 (s, 3H), 2.51 (s, 3H), 2.17 (s, 3H), 2.01 - 1.89 (m, 1H), 1.80-1.67 (m, 3H), 1.64-1.42 (m, 4H).

### Example 9 4-(4-Cyano-2-methoxyphenyl)-2,8-dimethyl-5-((1-methylcyclobutyl)methoxy) -1,4-dihydro-1,6-naphthyridine-3-carboxamide

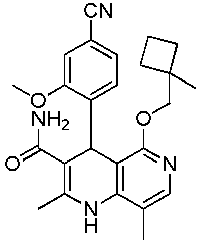

### Step 1) (1-methylcyclobutyl)methanol

1-Methylcyclobutane-1-carboxylic acid (0.67 mL, 6.57 mmol) was dissolved in tetrahydrofuran (20 mL) in a 100 mL single-neck flask under nitrogen protection and ice-bath condition, then to the mixture was added slowly lithium aluminium hydride (300 mg, 7.9 mmol). The mixture was heated to 35 °C and stirred for 4 hours. The mixture was cooled to room temperature, diluted with ethyl acetate (20 mL), and quenched with sodium sulfate hydrate (10 g). The mixture was filtered through a celite pad, and the filter cake was washed with ethyl acetate (30 mL × 2). The filtrate was concentrated *in vacuo* to remove the solvent to give light yellow oil (420 mg, 63.9 %).
¹H NMR (400 MHz, CDCl₃) δ (ppm) 3.47 (d, *J=* 2.0 Hz, 2H), 1.96 - 1.81 (m, 4H), 1.72 - 1.62 (m, 2H), 1.14 (s, 3H).

### Step 2) (1-Methylcyclobutyl)methyl methanesulfonate

(1-Methylcyclobutyl)methanol (420 mg, 4.2 mmol) and triethylamine (1.2 mL, 8.6 mmol) were dissolved in dichloromethane (20 mL). To the mixture was added methylsufonyl chloride (0.40 mL, 5.2 mmol). The mixture was stirred at room temperature overnight. To the mixture was added water (50 mL) to quench the reaction, and the resulting mixture was extracted with dichloromethane (50 mL × 2). The combined organic phases were washed with saturated saline (30 mL × 2), dried over anhydrous sodium sulfate, filtered, and the filtrate was concentrated *in vacuo* to give yellow oil (620 mg, 82.9 %).

### Step 3) Benzyl 4-(4-cyano-2-methoxyphenyl)-2,8-dimethyl-5-((1-methylcyclobutyl) methoxy)-1,4-dihydro-1,6-naphthyridine-3-carboxylate

Benzyl 4-(4-cyano-2-methoxyphenyl)-2,8-dimethyl-5-oxo-1,4,5,6-tetrahydro-1,6-naphthyridine-3-carboxylate (500 mg, 1.13 mmol), cesium carbonate (740 mg, 2.27 mmol) and (1-methylcyclobutyl)methyl methanesulfonate (400 mg, 2.24 mmol) were dissolved in *N*,*N*-dimethylformamide (20 mL). The mixture was stirred at 60 °C overnight. The mixture was cooled to room temperature, diluted with water (50 mL). The resulting mixture was extracted with ethyl acetate (50 mL × 2), and the organic layers were combined, washed with saturated brine (30 mL × 2). The organic layers were collected, evaporated under reduced pressure to remove the solvent, and the residue was purified by silica gel chromatography (petroleum ether /ethyl acetate (v/v) = 1/1) to give a light yellow solid (50 mg, 8.4%).
MS (ESI, pos. ion) m/z: 524.3 (M+1).

### Step 4) 4-(4-Cyano-2-methoxyphenyl)-2,8-dimethyl-5-((1-methylcyclobutyl)methoxy) -1,4-dihydro-1,6-naphthyridine-3-carboxylic acid

Benzyl 4-(4-cyano-2-methoxyphenyl)-2,8-dimethyl-5-((1-methylcyclobutyl)methoxy)-1,4 -dihydro-1,6-naphthyridine-3-carboxylate (60 mg, 0.12 mmol) and 10% Pd/C (12 mg) were added into tetrahydrofuran (20 mL). The mixture was stirred in hydrogen atomosphere overnight at 50 °C. The mixture was cooled to room temperature, filtered through a celite pad, then the solvent was evaporated under reduced pressure to give a white solid (48 mg, 96.6 %).

### Step 5) 4-(4-Cyano-2-methoxyphenyl)-2,8-dimethyl-5-((1-methylcyclobutyl)methoxy) -1,4-dihydro-1,6-naphthyridine-3-carboxamide

Ammonium chloride (30 mg, 0.56 mmol), 4-(4-Cyano-2-methoxyphenyl)-2,8-dimethyl -5-((1-methylcyclobutyl)methoxy)-1,4-dihydro-1,6-naphthyridine-3-carboxylic acid (50 mg, 0.12 mmol), O-(7-azabenzotriazol-1-yl)-*N*,*N*,*N'*,*N'*-tetramethyluronium hexafluorophosphate (90 mg, 0.24 mmol) and *N*,*N*-diisopropylethylamine (0.2 mL, 1 mmol) were dissolved in *N*,*N*-dimethylformamide (20 mL) under nitrogen protection. The resulting mixture was heated to 50 °C and stirred overnight. The mixture was cooled to room temperature, diluted with water (50 mL), and extracted with ethyl acetate (50 mL × 2), and the organic layers were combined, washed with saturated brine (30 mL × 2). The organic layers were collected, evaporated under reduced pressure to remove the solvent, and the residue was purified by silica gel column chromatography (dichloromethane/methanol(v/v) = 25/1) to give a white solid (10 mg, 20.05%).
MS (ESI, pos.ion) m/z: 433.2 (M+1);
¹H NMR (400 MHz, CDCl₃) δ (ppm) 7.71 (s, 1H), 7.30 (s, 1H), 7.16 (d, *J*= 7.9 Hz, 1H), 7.06 (s, 1H), 5.87 (s, 1H), 5.45 (s, 1H), 4.09 (d, *J=* 10.4 Hz, 1H), 4.00 (d, *J=* 10.4 Hz, 1H), 3.93 (s, 3H), 2.48 (s, 3H), 2.20 (s, 3H), 1.91 - 1.65 (m, 6H), 1.11 (s, 3H).

### Example 10 4-(4-Cyano-2-methoxyphenyl)-5-((1-cyanocyclopropyl)methoxy)-2,8-dimethyl-1,4-dihydro-1,6-naphthyridine-3-carboxamide

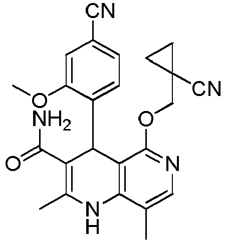

### Step 1) (1-Cyanocyclopropyl)methyl methanesulfonate

1-(Hydroxymethyl)cyclopropan-1-carbonitrile (500 mg, 5.15 mmol) and triethylamine (1.43 mL, 10.3 mmol) were dissolved in dichloromethane (20 mL). To the mixture was added methylsufonyl chloride (0.48 mL, 6.2 mmol). The mixture was stirred at room temperature overnight. To the mixture was added water (50 mL) to quench the reaction, and the resulting mixture was extracted with dichloromethane (50 mL × 2). The combined organic layers were washed with saturated saline (30 mL × 2). The organic layers were collected, evaporated *in vacuo* to remove the solvent and give yellow oil (900 mg, 99.78%).

### Step 2) Benzyl 4-(4-cyano-2-methoxyphenyl)-5-((1-cyanocyclopropyl)methoxy)-2,8 -dimethyl-1,4-dihydro-1,6-naphthyridine-3-carboxylate

Benzyl 4-(4-cyano-2-methoxyphenyl)-2,8-dimethyl-5-oxo-1,4,5,6-tetrahydro-1,6-naphthyridine-3-carboxylate (500 mg, 1.13 mmol), cesium carbonate (740 mg, 2.27 mmol) and (1-cyanocyclopropyl)methyl methanesulfonate (400 mg, 2.28 mmol) were dissolved in *N*,*N*-dimethylformamide (20 mL). The mixture was heated to 60 °C and stirred for 10 hours. The mixture was cooled to room temperature, diluted with water (50 mL). The resulting mixture was extracted with ethyl acetate (50 mL × 2), and the organic layers were combined, washed with saturated brine (30 mL × 2). The organic layers were collected, evaporated under reduced pressure to remove the solvent, and the residue was purified by silica gel column chromatography (petroleum ether/ethyl acetate (v/v) = 1/1) to give light yellow oil (240 mg, 40.71%).
MS (ESI, pos.ion) m/z: 521.2 (M+1).

### Step 3) 4-(4-Cyano-2-methoxyphenyl)-5-((1-cyanocyclopropyl)methoxy)-2,8-dimethyl -1,4-dihydro-1,6-naphthyridine-3-carboxylic acid

Benzyl 4-(4-cyano-2-methoxyphenyl)-5-((1-cyanocyclopropyl)methoxy)-2,8-dimethyl -1,4-dihydro-1,6-naphthyridine-3-carboxylate (240 mg, 0.46 mmol) and 10% Pd/C (40 mg) were added into tetrahydrofuran (20 mL). The mixture was stirred in hydrogen atomosphere overnight at room temperature. The reaction mixture was filtered through a celite pad, and the filtrate was evaporated under reduced pressure to remove the solvent. The residue was purified by silica gel column chromatography (petroleum ether/ethyl acetate (v/v) = 1/2) to give a white solid (80 mg, 40.31 %).
MS (ESI, pos.ion) m/z: 431.4 (M+1).

### Step 4) 4-(4-Cyano-2-methoxyphenyl)-5-((1-cyanocyclopropyl)methoxy)-2,8-dimethyl -1,4-dihydro-1,6-naphthyridine-3-carboxamide

Ammonium chloride (50 mg, 0.93 mmol), 4-(4-Cyano-2-methoxyphenyl)-5-((1-cyanocyclopropyl)methoxy)-2,8-dimethyl-1,4-dihydro-1,6-naphthyridine-3-carboxylic acid (80 mg, 0.19 mmol), O-(7-azabenzotriazol-1-yl)-*N*,*N*,*N*',*N*'-tetramethyluronium hexafluorophosphate (140 mg, 0.39 mmol) and *N*,*N*-diisopropylethylamine (0.2 mL, 1 mmol) were dissolved in *N*,*N*-dimethylformamide (20 mL) under nitrogen protection. The resulting mixture was heated to 50 °C and stirred overnight. The mixture was cooled to room temperature, diluted with water (50 mL), and extracted with ethyl acetate (50 mL × 2), and the organic layers were combined, washed with saturated brine (30 mL × 2). The organic layers were collected, evaporated under reduced pressure to remove the solvent, and the residue was purified by silica gel column chromatography (dichloromethane /methanol(v/v) = 25/1) to give a white solid (50 mg, 62.65 %).
MS (ESI, pos.ion) m/z: 430.2 (M+1);
¹H NMR (400 MHz, CDCl₃) δ (ppm) 7.66 (s, 1H), 7.32 (d, *J=* 7.9 Hz, 1H), 7.18 (d, *J*= 7.9 Hz, 1H), 7.10 (s, 1H), 5.80 (s, 1H), 5.46 (s, 1H), 5.07 - 4.99 (m, 1H), 4.02 (s, 3H), 2.52 (s, 3H), 2.46 - 2.37 (m, 1H), 2.25 - 2.19 (m, 1H), 2.17 (s, 3H), 2.03 - 1.92 (m, 1H), 1.75 - 1.68 (m, 1H), 1.65 - 1.55 (m, 2H).

### Example A Activity test in vitro

### Experimental principle:

Utilizing the characteristics of luciferase binding to the substrate to produce a chemiluminescence reaction, the fused plasmid of the Gal4 DNA binding domain (DBD) containing the ligand binding domain (LBD) of mineralocorticoid receptor (MR) and the firefly luciferase reporter gene plasmid controlled by Gal4 UAS (upstream activation sequences) were transfected into human embryonic kidney cells (HEK293). The changes in mineralocorticoid receptor activity between before and after stimulation or the influence of different stimuli on mineralocorticoid receptor activity was judged by the level of firefly luciferase activity. At the same time, in order to reduce the impact of internal change factors on the accuracy of the experiment, the plasmid with renilla luciferase gene was used as a control plasmid to transfect cells to provide an internal control for transcription activity, so that the test results were not interfered by changes in experimental conditions.

### Test method

1) HEK293 cells were collected after trypsinization and the cell density was adjusted to 500,000 cells/mL;
2) FuGENE HD transfection reagent was added into the cell suspension;
3) The above cell suspension was seeded into a 96-well cell culture plate at a density of 100 µL/well, and incubated for 24 hours at 37°C in 5% CO₂;
4) A series of concentrations of the test compound solution and the EC80 concentration of agonist aldosterone were added into each well and incubated for 18 hours;
5) Firefly and renilla luciferase signals were detected through Promega dual luciferase reporter assay system.

### Result processing:

1) After obtaining the firefly luciferase signal (F) and the Renilla luciferase signal (R), using the Renilla luciferase signal for correction, i.e., the F/R value was used to calculate the subsequent inhibition rate;
2)% inhibition rate = (Max-X) / (Max-Min) × 100%, where Max is the F/R value of the positive control well, Min is the F/R value of the negative control well, and X is the F/R value of test compound well of different concentrations;
3) IC₅₀ calculation was performed by GraphPrism 5.0 mapping software.

### Results were as shown below:

**Table 2 Activity in vitro of the compound of the invention**

| Example Number | MR IC₅₀ (nM) |
|---|---|
| Example 1 | 19.45 |
| Example 3 | 28.71 |
| Example 5 | 17.78 |
| Example 7 | 3.44 |
| Example 8 | 7.74 |

### Conclusion:

It can be seen from the experimental results in Table 2 that the compound of the present invention has good antagonistic activity against mineralocorticoid receptor (MR), and can be used as an effective mineralocorticoid receptor antagonist.

### Example B The pharmacokinetics test of the compound of the present invention

Preparation of the test compound solution: the test compound was formulated into a solution with 5% dimethyl sulfoxide, 5% Solutol HS 15 and 90% normal saline for oral and intravenous administration.

Male SD rats weighing 190-250 g were randomly grouped into two groups, each group had 3 numbers, one group was administered with test compound at a dose of 1.0 mg/kg by intravenous injection, and the other group was administered with test compound at a dose of 2.5 or 5.0 mg/kg by oral. After administering, blood samples were collected at time points of 0.0833, 0.25, 0.5, 1.0, 2.0, 4.0, 7 and 24 h. A standard curve in a suitable range was established based on concentrations of the samples, the concentrations of test compound in plasma samples in the MRM mode were determined by using AB SCIEX API4000 LC-MS/MS. Pharmacokinetic parameters were calculated according to drug concentration - time curve using a noncompartmental method by WinNonLin 6.3 software.

Experimental conclusion: the compound of the present invention has good pharmacokinetic properties in vivo.

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound having Formula (I) or a stereoisomer, a geometric isomer, a tautomer, an *N*-oxide, a hydrate, a solvate, a metabolite, an ester, a pharmaceutically acceptable salt or a prodrug thereof, 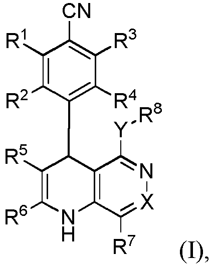
wherein X is CR^{x} or N;
each of R¹, R², R³ and R⁴ is independently H, D, amino, hydroxy, mercapto, cyano, nitro, C₁₋₆ alkyl, C₁₋₆ alkoxy, C₁₋₆ haloalkyl, C₁₋₆ haloalkoxy, C₁₋₆ alkylamino, carboxy, C₁₋₆ alkanoyl, C₁₋₆ alkylsulfonyl, aminocarbonyl, aminosulfonyl, C₃₋₈ cycloalkyl, C₆₋₁₀ aryl, 3-8 membered heterocyclyl or 5-10 membered heteroaryl;
R⁵ is cyano, -C(=O)R^{c} or -C(=O)NR^{a}R^{b};
R⁶ is H, D, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, 3-8 membered heterocyclyl or 5-10 membered heteroaryl;
each of R⁷ and R^{x} is independently H, D, halogen, cyano, C₁₋₆ alkoxycarbonyl, carboxy, C₁₋₆ alkyl, C₁₋₆ alkoxy, C₁₋₆ haloalkyl, C₁₋₆ haloalkoxy, C₁₋₆ alkylamino, C₁₋₆ alkanoyl, C₁₋₆ alkylsulfonyl, aminocarbonyl or aminosulfonyl;
each of R^{a} and R^{b} is independently H, D, C₁₋₆ alkyl or C₁₋₆ haloalkyl;
R^{c} is H, D, OH, C₁₋₆ alkoxy, C₁₋₆ alkyl, C₁₋₆ haloalkoxy, C₁₋₆ haloalkyl, C₆₋₁₀ aryl or 5-6 membered heteroaryl;
Y is O or S;
R⁸ is C₃₋₈ cycloalkyl, 3-8 membered heterocyclyl, 5-6 membered heteroaryl, C₃₋₈ cycloalkyl-C₁₋₆-alkyl, (3-8 membered heterocyclyl)-C₁₋₆ alkyl, (5-6 membered heteroaryl)-C₁₋₆ alkyl, phenyl or phenyl C₁₋₆ alkyl; wherein R⁸ is unsubstituted or substituted with 1, 2, 3 or 4 R^{z};
each R^{z} is independently =O, deuterium, fluorine, chlorine, bromine, iodine, hydroxy, cyano, NH₂, C₁₋₆ alkyl, C₁₋₆ alkoxy, C₁₋₆ haloalkoxy, C₁₋₆ haloalkyl, C₁₋₆ alkylamino, C₁₋₆ alkylsulfonyl, C₁₋₆ alkanoyl, C₃₋₈ cycloalkyl, 3-8 membered heterocyclyl, 5-6 membered heteroaryl or C₆₋₁₀ aryl; wherein, each R^{z} is independently unsubstituted or substituted with 1, 2, 3 or 4 R^{w};
each R^{w} is independently =O, deuterium, fluorine, chlorine, bromine, iodine, hydroxy, cyano, NH₂, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkanoyl, C₃₋₈ cycloalkyl, 3-8 membered heterocyclyl, 5-6 membered heteroaryl or C₆₋₁₀ aryl.

2. The compound of claim 1 having Formula (Ia) or Formula (Ib), or a stereoisomer, a geometric isomer, a tautomer, an *N*-oxide, a hydrate, a solvate, a metabolite, an ester, a pharmaceutically acceptable salt or a prodrug thereof,

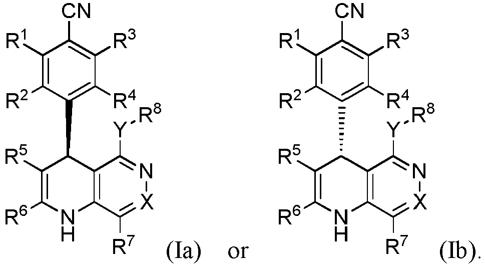

3. The compound of any one of claims 1 to 2, wherein each of R¹, R², R³ and R⁴ is independently H, D, amino, hydroxy, mercapto, cyano, nitro, C₁₋₄ alkyl, C₁₋₄ alkoxy, C₁₋₄ haloalkyl, C₁₋₄ haloalkoxy, C₁₋₄ alkylamino, carboxy, C₁₋₄ alkanoyl, C₁₋₄ alkylsulfonyl, aminocarbonyl, aminosulfonyl, C₃₋₆ cycloalkyl, C₆₋₁₀ aryl, 3-6 membered heterocyclyl or 5-6 membered heteroaryl;
R⁶ is H, D, C₁₋₄ alkyl, C₁₋₄ alkoxy, C₁₋₄ haloalkyl, C₁₋₄ haloalkoxy, C₁₋₄ alkylamino, C₃₋₆ cycloalkyl, C₆₋₁₀ aryl, 3-6 membered heterocyclyl or 5-6 membered heteroaryl;
each of R⁷ and R^{x} is independently H, D, halogen, cyano, C₁₋₄ alkoxycarbonyl, carboxy, C₁₋₄ alkyl, C₁₋₄ alkoxy, C₁₋₄ haloalkyl, C₁₋₄ haloalkoxy, C₁₋₄ alkylamino, C₁₋₄ alkanoyl, C₁₋₄ alkylsulfonyl, aminocarbonyl or aminosulfonyl;
each of R^{a} and R^{b} is independently H, D, C₁₋₄ alkyl or C₁₋₄haloalkyl.

4. The compound of any one of claims 1 to 3, wherein each of R¹, R², R³ and R⁴ is independently H, D, amino, hydroxy, mercapto, cyano, nitro, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethoxy, difluoromethoxy, monofluoromethoxy, methylamino, dimethylamino, carboxy, methylcarbonyl, ethylcarbonyl, methylsulfonyl, aminocarbonyl or aminosulfonyl;
each of R^{a} and R^{b} is independently H, D, methyl, ethyl, propyl, butyl, trifluoromethyl, difluoromethyl, monofluoromethyl, 2,2-difluoroethyl, 1,2-difluoroethyl or trifluoroethyl;
R⁶ is H, D, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethoxy, difluoromethoxy, monofluoromethoxy, methylamino, dimethylamino, cyclopropyl, cyclobutyl, cyclopentyl, cyclohexyl, phenyl, naphthyl, epoxyethyl, pyrrolidyl, piperidyl, piperazinyl, morpholinyl, pyridyl, pyrrolyl, thiazolyl, pyrazolyl or pyrimidinyl;
R⁷ is H, D, cyano, methylcarbonyl, ethylcarbonyl, propylcarbonyl, methoxycarbonyl, ethoxycarbonyl, propoxycarbonyl, carboxy, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethoxy, difluoromethoxy, monofluoromethoxy, methylamino or dimethylamino.

5. The compound of any one of claims 1 to 4 having Formula (IIIa) or Formula (IIIb), or a stereoisomer, a geometric isomer, a tautomer, an *N*-oxide, a hydrate, a solvate, a metabolite, an ester, a pharmaceutically acceptable salt or a prodrug thereof,

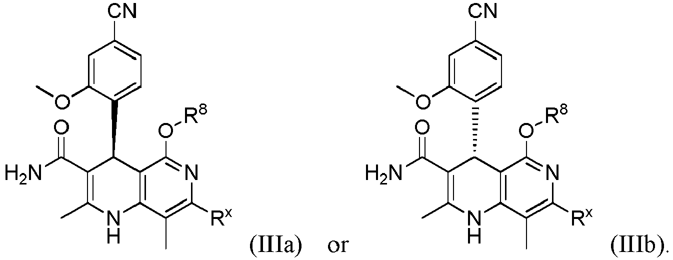

6. The compound of any one of claims 1 to 5, wherein R^{x} is H, D, fluorine, chlorine, bromine, iodine, cyano, methylcarbonyl, ethylcarbonyl, propylcarbonyl, methoxycarbonyl, ethoxycarbonyl, propoxycarbonyl, carboxy, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethoxy, difluoromethoxy, monofluoromethoxy, methylamino or dimethylamino.

7. The compound of any one of claims 1 to 6, wherein R⁸ is C₃₋₆ cycloalkyl, 3-6 membered heterocyclyl, 5-6 membered heteroaryl, C₃₋₆ cycloalkyl-C₁₋₄-alkyl, (3-6 membered heterocyclyl)-C₁₋₄ alkyl or (5-6 membered heteroaryl)-C₁₋₄ alkyl; wherein R⁸ is unsubstituted or substituted with 1, 2, 3 or 4 R^{z}.

8. The compound of any one of claims 1 to 7, wherein R⁸ is cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl, cyclopropylmethyl, cyclopropylethyl, cyclobutylmethyl, cyclobutylethyl, cyclopentylmethyl, cyclopentylethyl, cyclohexylmethyl, cyclohexylethyl, azetidinylmethyl, azetidinylethyl, oxetanylmethyl, oxetanylethyl, pyrrolidylmethyl, pyrrolidylethyl, tetrahydrofurylmethyl, tetrahydrofurylethyl, tetrahydrothienylmethyl, tetrahydrothienylethyl, piperidinylmethyl, piperidinylethyl, piperazinylmethyl, piperazinylethyl, morpholinylmethyl, morpholinylethyl, pyrrolylmethyl, pyrrolylethyl, furylmethyl, furylethyl, thienylmethyl, thienylethyl, thiazolylmethyl, thiazolylethyl, pyrazolylmethyl, pyrazolylethyl, imidazolylmethyl, imidazolylethyl, triazolylmethyl, triazolylethyl, tetrazolylmethyl, tetrazolylethyl , pyridylmethyl, pyridylethyl, pyrimidinylmethyl or pyrimidinylethyl; wherein R⁸ is unsubstituted or substituted with 1, 2, 3 or 4 R^{z}.

9. The compound of any one of claims 1 to 8, wherein each R^{z} is independently =O, deuterium, fluorine, chlorine, bromine, iodine, hydroxy, cyano, NH₂, C₁₋₄ alkyl, C₁₋₄ alkoxy, C₁₋₄ haloalkoxy, C₁₋₄ haloalkyl, C₁₋₄ alkylamino, C₁₋₄ alkylsulfonyl, C₁₋₄ alkylcarbonyl, C₃₋₆ cycloalkyl, 3-6 membered heterocyclyl, 5-6 membered heteroaryl or C₆₋₁₀ aryl; wherein, each R^{z} is independently unsubstituted or substituted with 1, 2, 3 or 4 R^{w};
each R^{w} is independently =O, deuterium, fluorine, chlorine, bromine, iodine, hydroxy, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkanoyl, C₃₋₆ cycloalkyl, 3-6 membered heterocyclyl, 5-6 membered heteroaryl or C₆₋₁₀ aryl.

10. The compound of any one of claims 1-9, wherein each R^{z} is independently =O, deuterium, fluorine, chlorine, bromine, iodine, hydroxy, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino , ethylamino, dimethylamino, methylethylamino, diethylamino, methylsulfonyl, ethylsulfonyl, methylcarbonyl, ethylcarbonyl, cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl or phenyl; wherein each R^{z} is independently unsubstituted or substituted with 1, 2, 3 or 4 R^{w};
each R^{w} is independently =O, deuterium, fluorine, chlorine, bromine, iodine, hydroxy, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methylethylamino, diethylamino, methylsulfonyl, ethylsulfonyl, methylcarbonyl, ethylcarbonyl, cyclopropyl, cyclobutyl, cyclopentyl, cyclohexyl, oxiranyl, azetidinyl, oxetanyl, pyrrolidyl, tetrahydrofuryl, tetrahydrothienyl, thiazolidyl, pyrazolidyl, oxazolidyl, imidazolidyl, isoxazolidyl, piperidyl, piperazinyl, morpholinyl, pyrrolyl, furyl, thienyl, thiazolyl, pyrazolyl, pyridyl, pyrimidinyl or phenyl.

11. The compound of any one of claims 1 to 10 having one of the following structures, or a stereoisomer, a geometric isomer, a tautomer, an *N*-oxide, a hydrate, a solvate, a metabolite, an ester, a pharmaceutically acceptable salt or a prodrug thereof,

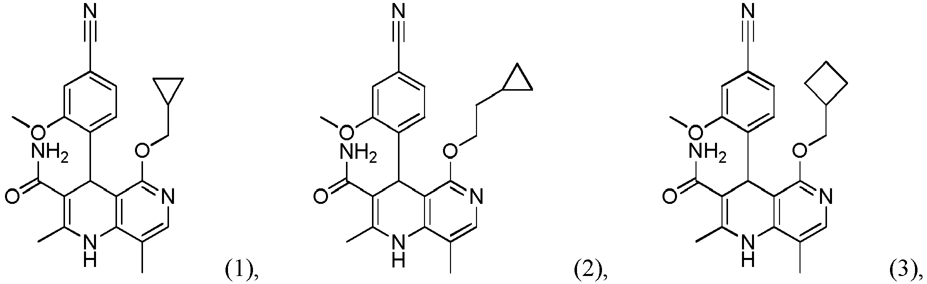

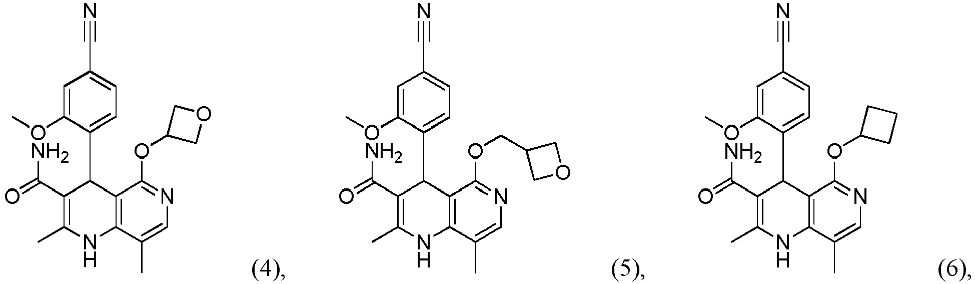

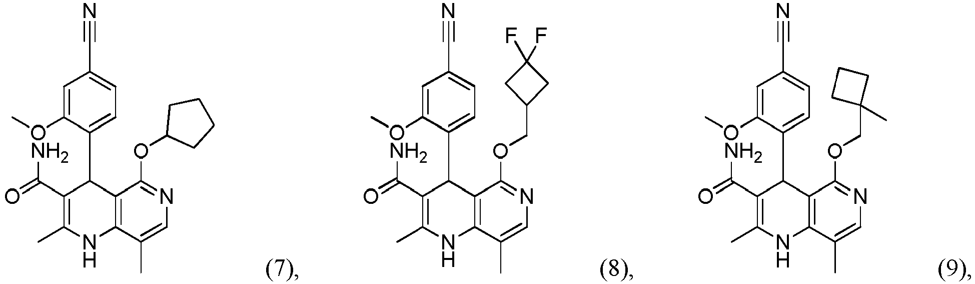

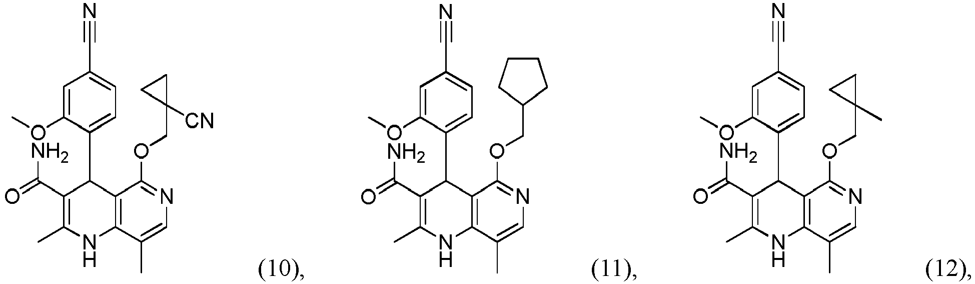

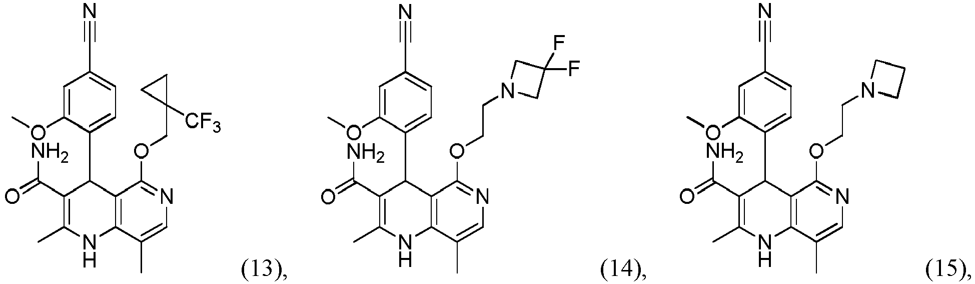

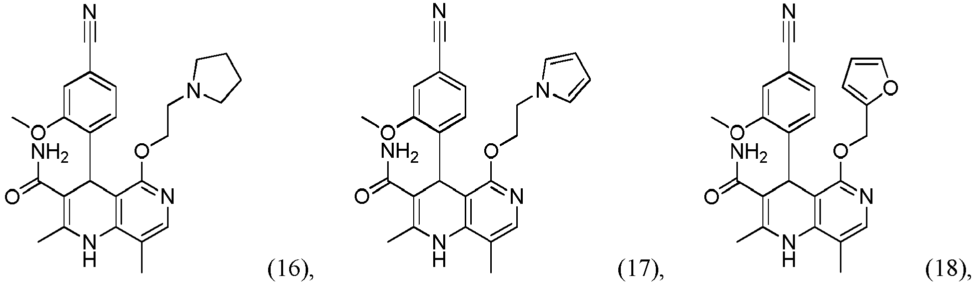

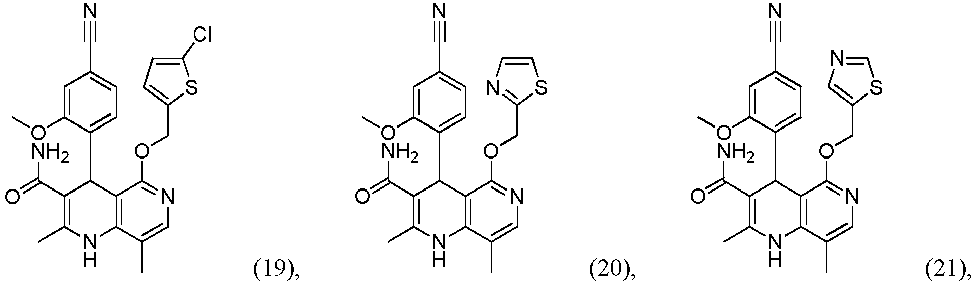

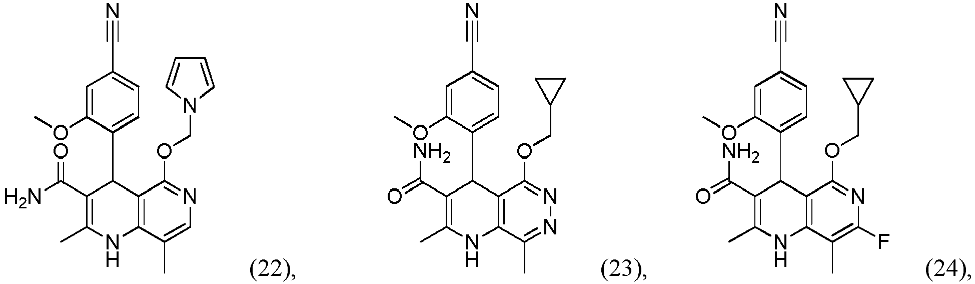

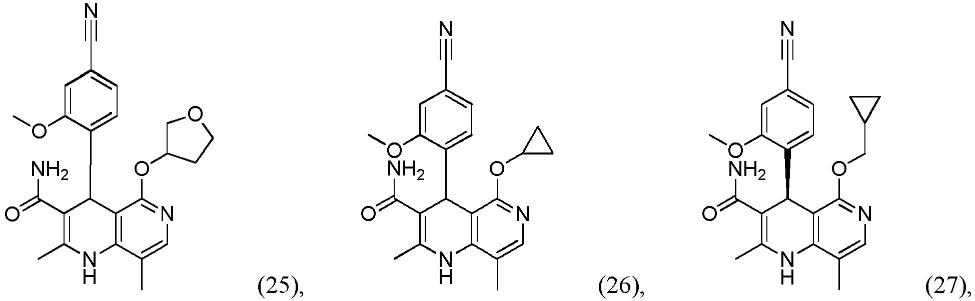

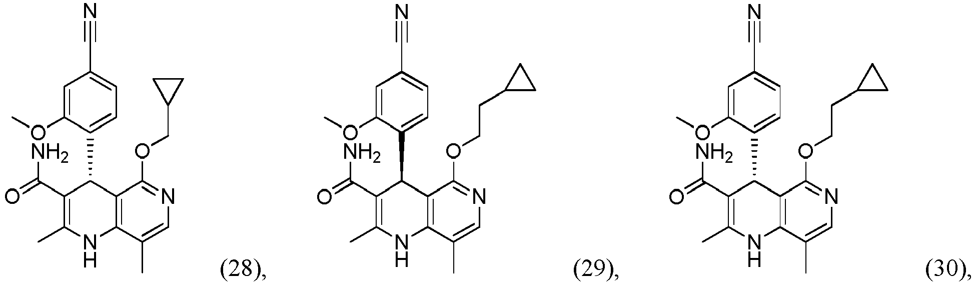

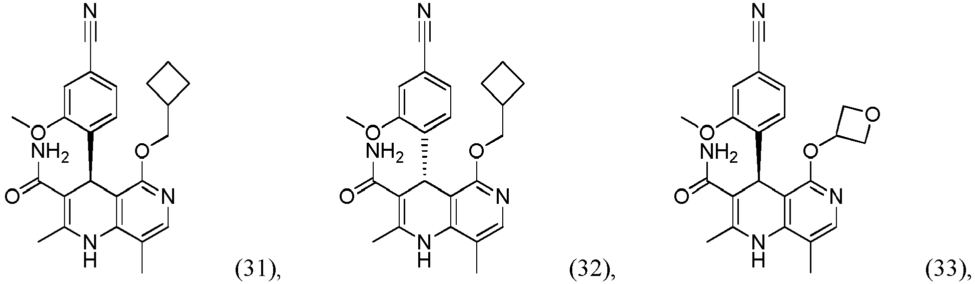

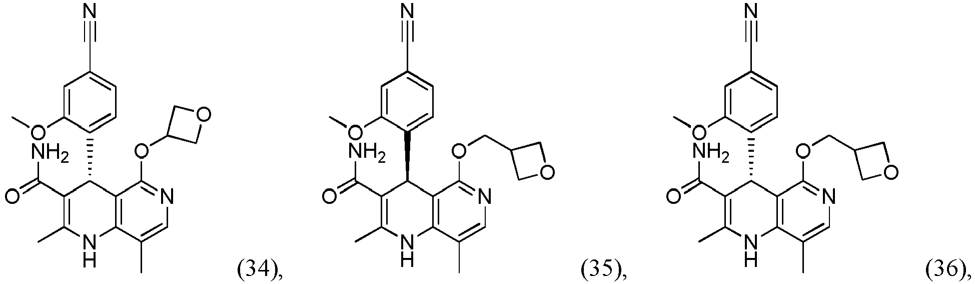

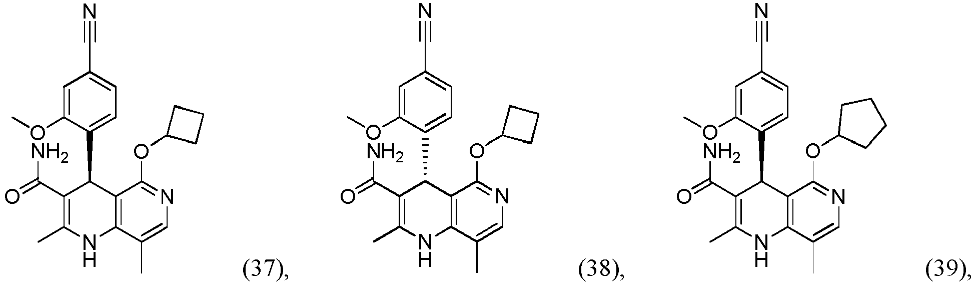

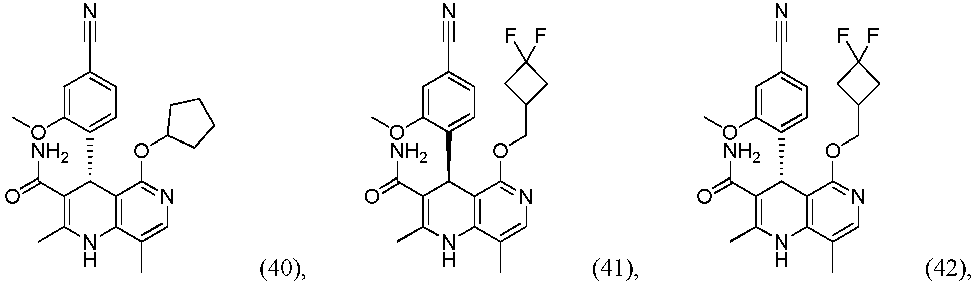

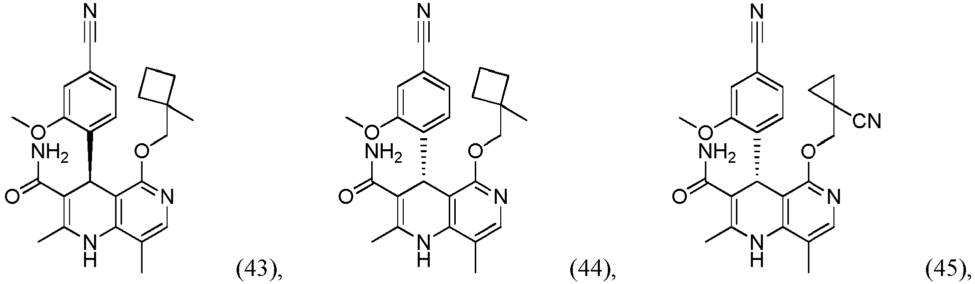

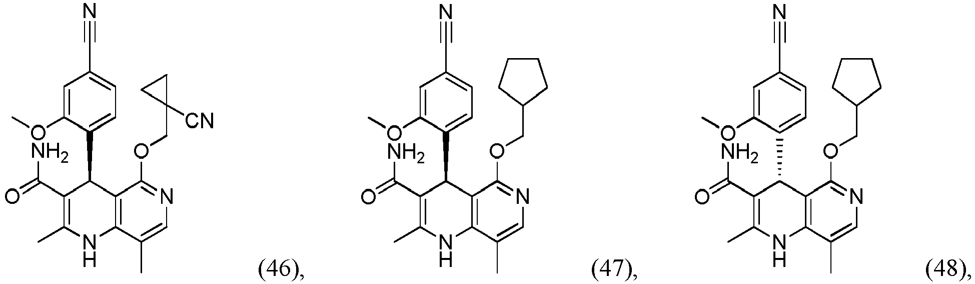

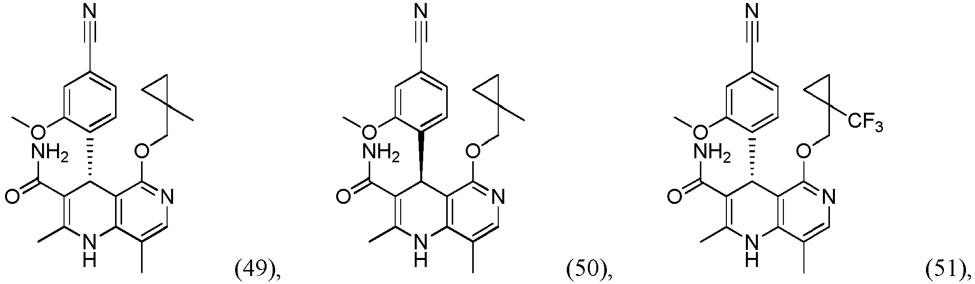

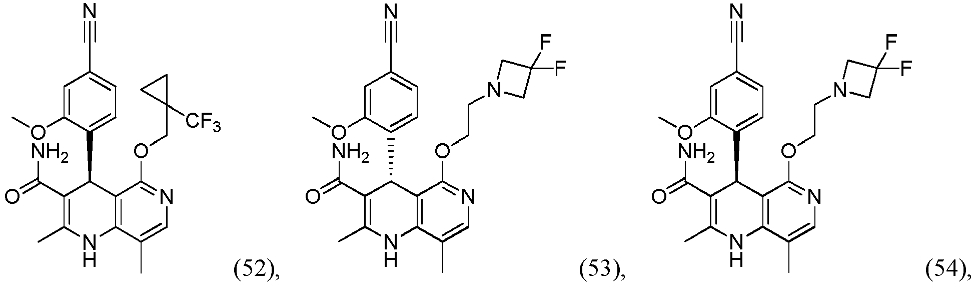

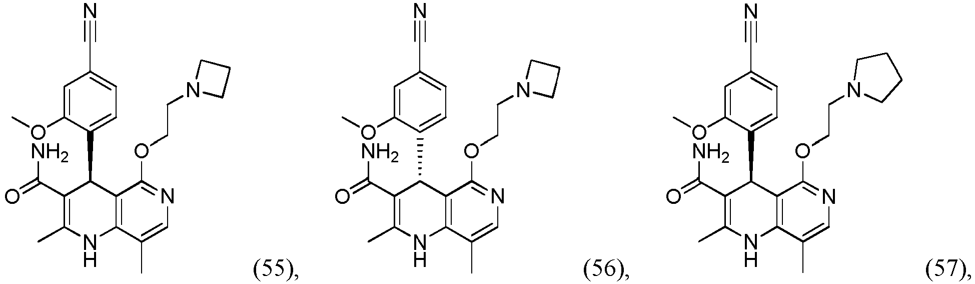

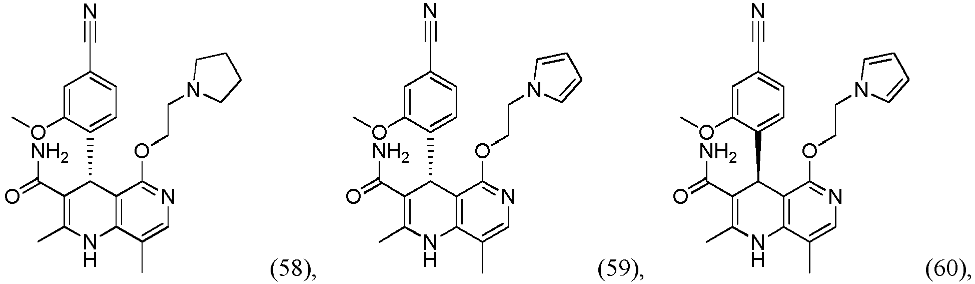

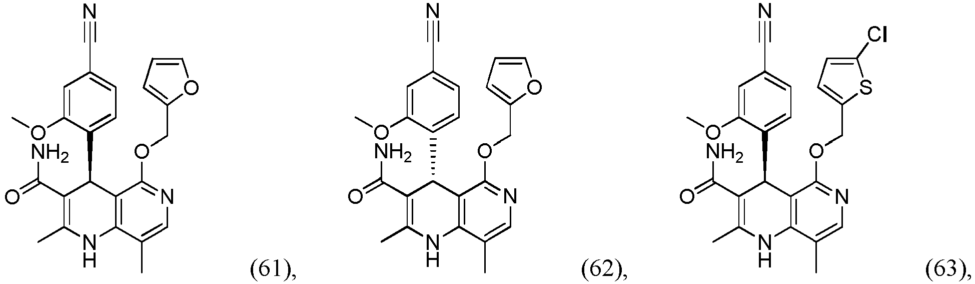

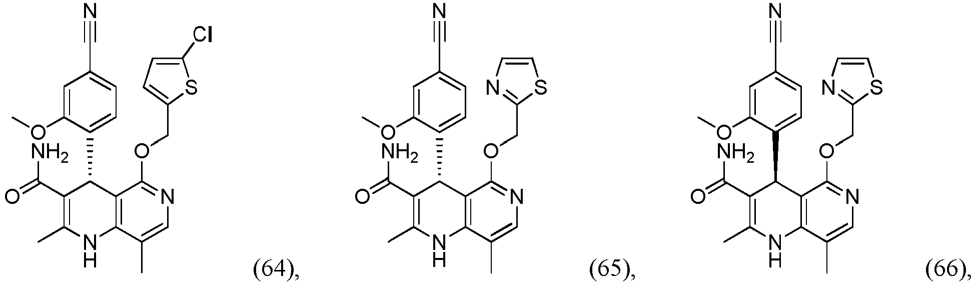

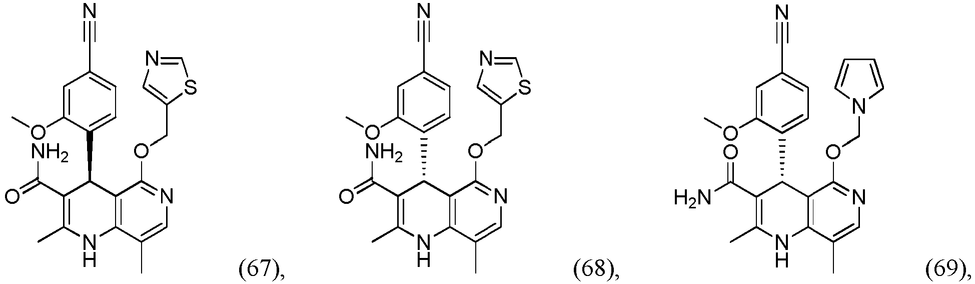

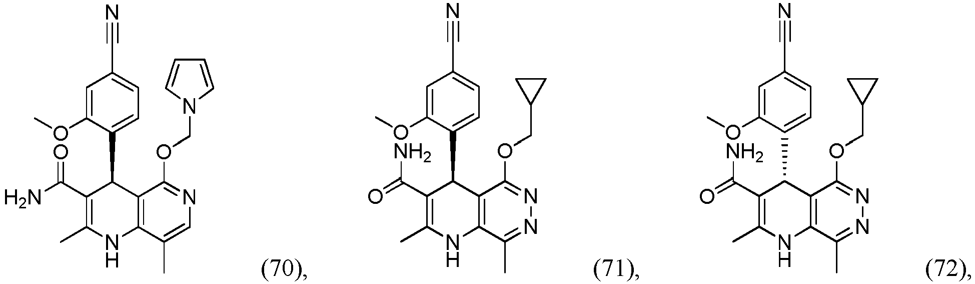

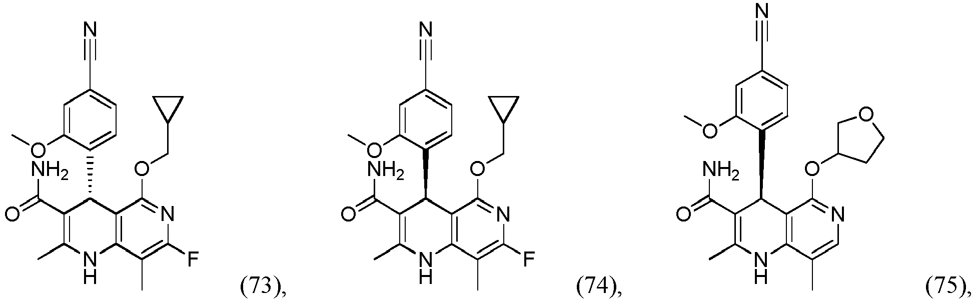

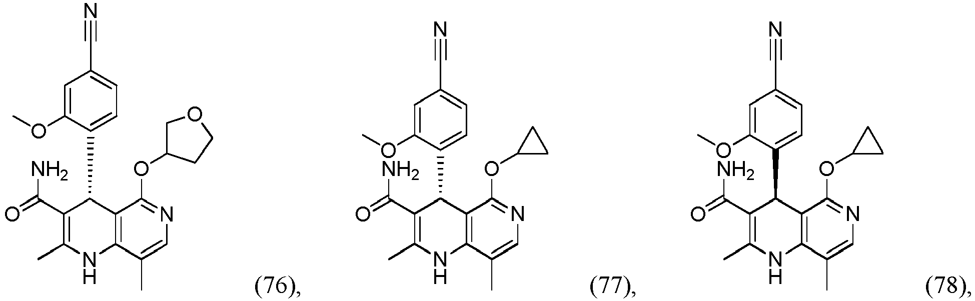

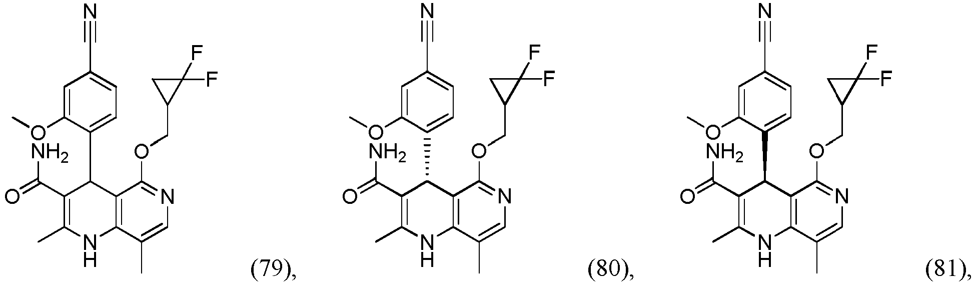

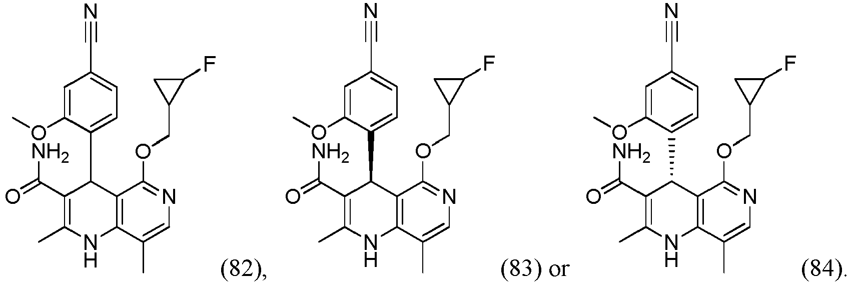

12. A pharmaceutical composition comprising the compound of any one of claims 1 to 11; and optionally, further comprising a pharmaceutically acceptable carrier, excipient, diluent, adjuvant, or a combination thereof.

13. The pharmaceutical composition of claim 12 further comprising one or more other active ingredients selected from ACE inhibitors, renin inhibitors, angiotensin II receptor antagonists, β-receptor blockers, acetylsalicylic acid, diuretics, calcium antagonists, statins, digitalis derivatives, calcium sensitizers, nitrates and antithrombotic agents.

14. Use of the compound of any one of claims 1 to 11 or the composition of any one of claims 12 to 13 in the manufacture of a medicament for treating, preventing or lessening the following diseases: diabetic nephropathy, hyperaldosteronism, hypertension, heart failure, sequelae of myocardial infarction, liver cirrhosis, renal failure or stroke.

15. Use of the compound of any one of claims 1 to 11 or the composition of any one of claims 12 to 13 in the manufacture of a medicament as a mineralocorticoid receptor antagonist.

16. The compound of any one of claims 1 to 11 or the composition of any one of claims 12 to 13 for use in treating, preventing or lessening the following diseases in a patient: diabetic nephropathy, hyperaldosteronism, hypertension, heart failure, sequelae of myocardial infarction, liver cirrhosis, renal failure or stroke.

17. The compound of any one of claims 1 to 11 or the composition of any one of claims 12 to 13 for use in antagonizing mineralocorticoid receptor.

18. A method of treating, preventing or lessening the following diseases in a patient: diabetic nephropathy, hyperaldosteronism, hypertension, heart failure, sequelae of myocardial infarction, liver cirrhosis, renal failure or stroke, comprising administering to the patient the compound of any one of claims 1 to 11 or the composition of any one of claims 12 to 13 .

19. A method of using the compound of any one of claims 1 to 11 or the composition of any one of claims 12 to 13 in antagonizing mineralocorticoid receptor.
